# EUROPEAN PATENT SPECIFICATION

(11) **EP 4 547 663 B1**
(45) Date of publication and mention of the grant of the patent: **24.06.2026**
(21) Application number: 23736306.4
(22) Date of filing: 29.06.2023
(51) Int. Cl.: C07D 471/04, A61P 19/02, A61P 3/10, A61P 1/16, A61P 29/00

(54) **IMIDAZO[4,5-C]PYRIDINE DERIVATIVES AS SIK MODULATORS FOR THE TREATMENT OF RHEUMATOID ARTHRITIS**
IMIDAZO[4,5-C]PYRIDINE-DERIVATE ALS SIK MODULATOREN ZUR BEHANDLUNG VON RHEUMATOIDER ARTHRITIS
DÉRIVÉS D'IMIDAZO[4,5-C]PYRIDINE EN TANT QUE MODULATEURS DE SIK POUR LE TRAITEMENT DE POLYARTHRITE RHUMATOÏDE

(30) Priority: 01.07.2022 EP 22182508
(43) Date of publication of application: 07.05.2025
(73) Proprietor: F. Hoffmann-La Roche AG, 4070 Basel (CH)
(72) Inventor: DÉCORET, Guillaume, 4070 Basel (CH); GROEBKE ZBINDEN, Katrin, 4070 Basel (CH); HAAP, Wolfgang, 4070 Basel (CH); KREIS, Lukas, 4070 Basel (CH); LUCAS CABRÉ, Xavier, 4070 Basel (CH); RAUBER, Etienne, 4070 Basel (CH)
(74) Representative: Vitra, Hermeto
(86) International application number: PCT/EP2023/067759
(87) International publication number: WO 2024/003209

(56) References cited:
- WO-A1-2020/239658

## Description

The present invention relates to organic compounds useful for therapy and/or prophylaxis in a mammal, and in particular to compounds that modulate SIK activity.

The invention relates in particular to a compound of formula (I) 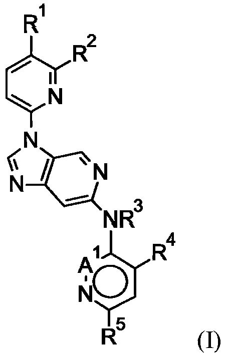 wherein
R¹ is hydroxyalkyl, alkylcarbonyl, cyclopropyl, haloalkyl or cyano;
R² is heteroaryl optionally substituted with one, two or three substituents independently selected from alkyl, haloalkyl, halogen, cyano, haloalkoxy and alkoxy;
R³ is hydrogen or alkyl;
R⁴ is hydrogen or alkyl; or R³ together with R⁴ and the atoms they are attached to, form a five- to seven-membered heterocycloalkyl or heteroaryl ring;
R⁵ is alkyl or haloalkyl; and
A¹ is -CH- or -N-;
or a pharmaceutically acceptable salt thereof.

Salt-inducible kinases (SIK) belong to a subfamily of AMP-activated protein kinases (AMPK) called AMPK-related kinases. There are three members, named SIK1, SIK2 and SIK3, that are broadly expressed. Their major biological role is to modify gene expression by controlling the phosphorylation and subcellular localization of two key classes of transcriptional regulatory factors: CRTCs (cAMP-regulated transcriptional coactivators) and class IIa HDACs (Histone deacetylases). Indeed, in basal state, both CRTCs and HDACs are phosphorylated by SIK kinases, and sequestered in the cytoplasm through interactions with their cytoplasmic chaperones 14-3-3. In response to extracellular cues that usually increase intracellular levels of cAMP, the SIK kinases' activity is inhibited, CRTCs and HDACs are no longer phosphorylated and are hence released from 14-3-3. They can therefore translocate into the nucleus and regulate gene expression (reviewed in Wein et al., Trends Endocrinol Metab. 2018 Oct;29(10):723-735).

In macrophages, the inhibition of SIK kinases leads to 1) CRTC3 shuttling to the nucleus and increasing the transcription of IL-10,; and 2) translocation of HDAC4/5 to the nucleus and subsequent deacetylation of NF-κB resulting in decreased transcription of pro-inflammatory cytokines (Clark et al., Proc Natl Acad Sci U S A. 2012 Oct 16;109(42):16986-91.).

Macrophages are critical to maintaining tissue homeostasis, mediating inflammation, and promoting the resolution of inflammation. To achieve this diversity of function, macrophages have the ability to "polarize" differently in response to environment cues. The two extreme phenotypes along their activation state continuum are the "M1" or "pro-inflammatory macrophages" and the "M2" or "pro-resolution macrophages".

Strikingly, the inhibition of intracellular SIK kinases overrides these extracellular macrophage polarization signals and pushes them toward a pro-resolution phenotype. This comes with an increase in IL-10 (by interfering with the SIK-CRTC3 pathway) and a concomitant decrease in TNF-α, IL-12 and IL-6 (by interfering with the SIK-HDAC4/5 and NF-κB pathway). The high levels of IL-10 and low levels of pro-inflammatory cytokines upon SIK inhibition will promote resolution of inflammation. The exploration of the SIK pathway has initially been described in macrophages (Clark et al., Proc Natl Acad Sci U S A. 2012 Oct 16;109(42):16986-91) and dendritic cells (Sundberg et al., Proc Natl Acad Sci U S A. 2014 Aug 26;111(34):12468-73) and the therapeutic potential of pan-SIK inhibitors has been confirmed in a mouse LPS (lipopolysaccharide) challenge model (Sundberg et al., ACS Chem Biol. 2016 Aug 19;11(8):2105-11) and in colitis models (Fu et al., Inflamm Bowel Dis. 2021 Oct 20;27(11):1821-1831). SIKs have since been shown to be important players in the functions of several immune cells, including mast cells (Darling et al., J Biol Chem. 2021 Jan-Jun;296:100428). Importantly, SIK1 is poorly expressed in macrophages and one embodiment of the invention are SIK2/3 inhibitors sparing SIK1, thus limiting potential SIK1-related toxicities.

SIK inhibitors have a high therapeutic potential in diseases that are 1) characterized by pro-inflammatory macrophage influx in the tissues and impaired tissue homeostasis and healing, or 2) where anti-TNF therapies are beneficial (partially or fully) or with insufficient levels of the IL10. Diseases with an inflammatory macrophage signature are e.g. rheumatoid arthritis, juvenile rheumatoid arthritis, NASH, primary sclerosing cholangitis, giant cell vasculitis and inflammatory bowel diseases ("IBD"), atherosclerosis, type 2 diabetes and glomerulonephritis.

Diseases with a proven link to IL-10 and TNF-α are IBD. Genetic alterations that reduce the function of IL-10 (such as SNPs in IL-10 or its receptor) are associated with an increased risk for IBD in humans. In addition, anti-TNF therapies are successful but only a subset of IBD patients are responsive and much of this limited responsiveness is lost over time. The described dual effect of SIK inhibitors (increased IL-10 and decreased TNF-α) make them particularly pertinent for the treatment of IBD.

All three SIK kinase isoforms are expressed broadly in human tissues with the highest expression observed in skin and adipose tissues for SIK1, adipose tissue for SIK2 and testis and brain for SIK3. Similarly to their role in macrophages, SIKs in these cells phosphorylate CRTCs and class II HDACs in response to extracellular signals, which subsequently change the expression of several cellular factors.

In addition to their physiological roles, reports have linked dysregulation of SIK expression to a few diseases. For example, SIK2 has been described as a risk locus for primary sclerosing cholangitis, a fibrotic disease regularly associated with IBD. In addition, SIK2 and SIK3 expression is higher in ovarian and prostate cancers and correlated with poor survival (Miranda et al., Cancer Cell. 2016 Aug 8;30(2):273-289; Bon et al., Mol Cancer Res. 2015 Apr;13(4):620-635).

As of today many diseases caused by dysregulation of the innate immune system lack efficient therapies and there is a high unmet medical need for new therapies. The present invention relates to a novel compounds that are highly active SIK inhibitors for the treatment of inflammatory, allergic and autoimmune diseases. In addition to inflammation, allergic and autoimmune diseases, SIK inhibitors can thus also be of potential relevance in cancer, metabolic diseases, bone density dysregulation diseases, pigmentation-related diseases or cosmetology, fibrotic diseases and depressive disorders.

WO 2020/239658 A1 discloses fused N-heterocyclic compounds, such as e.g. imidazo[1,2-a]pyridine derivatives, as SIK modulators for the treatment of e.g. rheumatoid arthritis.

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C1-C8 alkyl groups include, but are not limited to, methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl, propyl, isopropyl, butyl and isobutyl. Methyl, ethyl, propyl and butyl, like isobutyl, are further particular examples of "alkyl" in the compound of formula (I).

The term "cycloalkyl", alone or in combination, signifies a cycloalkyl ring with 3 to 8 carbon atoms and particularly a cycloalkyl ring with 3 to 6 carbon atoms. Examples of cycloalkyl include, but are not limited to, cyclopropyl, cyclobutyl, cyclopentyl, cyclohexyl, cycloheptyl and cyclooctyl. Particular examples of "cycloalkyl" are cyclopropyl and cyclobutyl.

The term "heterocycloalkyl", alone or in combination, denotes a monovalent saturated or partly unsaturated mono- or bicyclic ring system of 4 to 12 ring atoms, comprising 1, 2, or 3 ring heteroatoms selected from N, O and S, the remaining ring atoms being carbon. Bicyclic means consisting of two cycles having one or two ring atoms in common. "Hetercycloylkyl" may comprise a carbonyl group, wherein the carbon is part of the ring system. The ring system can be attached to the remaining compound via an atom selected from C, N, S and O, in particular via a N atom ("N-heterocycloalkyl). Examples of "heterocycloalkyl" include, but are not limited to, morpholino, morpholin-4-yl, pyrrolidinyl, pyrrolidin-1-yl, pyrrolidin-3-yl, piperidinyl, 1-piperidyl, 4-piperidyl, 2-oxopyrrolidin-1-yl, piperazinyl, piperazin-1-yl, azetidinyl, azetidin-1-yl, [3-oxo-piperazin-1-yl], (1,1-dioxo-1,2-thiazolidin-2-yl), (4,5,6,7-tetrahydropyrazolo[4,3-c]pyridin-1-yl), (3-oxo-1,5,6,8-tetrahydrooxazolo[3,4-a]pyrazin-7-yl), [rac-(3aR,6aS)-2,3,3a,5,6,6a-hexahydro-1H-pyrrolo[3,2-b]pyrrol-4-yl], [rac-(3aS,6aR)-2,3,3a,5,6,6a-hexahydro-1H-pyrrolo[3,2-b]pyrrol-4-yl], (4-oxo-6,7-dihydro-5H-pyrazolo[1,5-a]pyrazin-3-yl), (6,7-dihydro-4H-pyrazolo[4,3-c]pyridin-1-yl), (4,7-diazaspiro[2.5]octan-7-yl), (2-oxa-5,8-diazaspiro[3.5]nonan-8-yl), 3-azabicyclo[3.2.0]heptan-3-yl), (5-azaspiro[2.4]heptan-5-yl), (2-azabicyclo[2.2.1]heptan-2-yl), 4-oxa-7-azaspiro[2.5]octan-7-yl, (3-azabicyclo[3.1.0]hexan-3-yl), (6,7-dihydro-4H-pyrazolo[4,3-c]pyridin-1-yl), 2-oxa-7-azaspiro[3.4]octan-7-yl, (2-oxo-1-piperidyl), (2,3-dihydropyridazino[4,5-b][1,4]oxazin-8-yl), pyrrolidin-1-yl, 2-oxo-pyrimidin-4-yl, morpholinoethyl, 2-oxa-5-azaspiro[3.4]octan-5-yl, oxetan-3-yl, (2-oxo-1-piperidyl), 2-oxo-4-piperidyl, 5-oxo-pyrrolidin-3-yl, 2-oxa-5-azaspiro[3.4]octan-5-yl, (7,8-dihydro-5H-pyrano[4,3-c]pyridazin-3-yl), [rac-(4aS,7aR)-4-methyl-2,3,4a,5,7,7a-hexahydropyrrolo[3,4-b][1,4]oxazin-6-yl], 3,4-dihydro-2H-1,4-oxazinyl and [rac-(3aS,6aS)-6-oxo-2,3,3a,4,5,6a-hexahydropyrrolo[2,3-c]pyrrol-1-yl]. A particular example of "heterocycloalkyl" is 3,4-dihydro-2H-1,4-oxazine.

The term "heteroaryl", alone or in combination, signifies an aromatic mono- or bicyclic ring system with 5 to 12 ring atoms, comprising 1, 2, 3 or 4 heteroatoms each independently selected from N, O and S, the remaining ring atoms being carbon. The ring system can be attached to the remaining compound via an atom selected from C, N, S and O, in particular via a N atom ("N-heteroaryl). Examples of heteroaryl include, but are not limited to, pyrazolyl, pyrazol-1-yl, pyrazol-3-yl, pyrazol-4-yl, pyridinyl, 2-pyridyl, 3-pyridyl, 4-pyridyl, pyridazinyl, pyridazin-3-yl, pyridazin-4-yl, pyrazinyl, pyrazin-2-yl, isoxazolyl, isoxazol-3-yl, isoxazol-4-yl, pyrimidinyl, pyrimidin-5-yl, benzotriazolyl, 1H-benzotriazol-4-yl, furanyl, furyl, 2-furyl, 3-furyl, [6-oxo-1H-pyridazin-5-yl], triazolyl, triazol-1-yl, triazol-2-yl, 2-oxo-4-pyridyl. pyrimidin-2-yl, pyrimidin-5-yl, (1,3,4-oxadiazol-2-yl), (1,3,4-thiadiazol-2-yl), (1,2,4-triazin-3-yl), 2-oxo-pyrimidin-4-yl, (1-methyl-2-oxo-3-pyridyl) and (2,3-dihydropyridazino[4,5-b][1,4]oxazin-8-yl). Particular examples of "heteroaryl" are pyrazol-1-yl, pyrazol-4-yl, pyridazin-3-yl ane pyrimidin-5-yl.

The term "alkoxy" or "alkyloxy", alone or in combination, signifies a group of the formula alkyl-O- in which the term "alkyl" has the previously given significance, such as methoxy, ethoxy, n-propoxy, isopropoxy, n-butoxy, isobutoxy, sec-butoxy and tert.-butoxy. Particular examples of "alkoxy" are methoxy and ethoxy.

The term "oxy", alone or in combination, signifies the -O- group.

The term "oxo", alone or in combination, signifies the =O group.

The terms "halogen" or "halo", alone or in combination, signifies fluorine, chlorine, bromine or iodine and particularly fluorine, chlorine or bromine, more particularly fluorine. The term "halo", in combination with another group, denotes the substitution of said group with at least one halogen, particularly substituted with one to five halogens, particularly one to four halogens, i.e. one, two, three or four halogens.

The term "haloalkyl", alone or in combination, denotes an alkyl group substituted with at least one halogen, particularly substituted with one to five halogens, particularly one to three halogens, more particularly two to three halogens. Particular "haloalkyl" are fluoromethyl, fluoroethyl, fluoropropyl, fluorobutyl, difluoromethyl, difluoroethyl, trifluoromethyl and trifluoroethyl.

The term "haloalkoxy", alone or in combination, denotes an alkoxy group substituted with at least one halogen, particularly substituted with one to five halogens, particularly one to three halogens. Particular "haloalkoxy" are fluoromethoxy, fluoroethoxy and fluoropropyloxy.

The terms "hydroxyl" and "hydroxy", alone or in combination, signify the -OH group.

The term "carbonyl", alone or in combination, signifies the -C(O)- group.

The term "amino", alone or in combination, signifies the primary amino group (-NH₂), the secondary amino group (-NH-), or the tertiary amino group (-N-).

The term "alkylamino" is alkyl group linked to a -NH- group. The term "dialkylamino" denotes two alkyl groups linked to a -N- atom.

The term "sulfonyl", alone or in combination, signifies the -SO₂- group.

The term "pharmaceutically acceptable salts" denotes salts which are not biologically or otherwise undesirable. Pharmaceutically acceptable salts include both acid and base addition salts. The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid. The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, and polyamine resins. Particular pharmaceutically acceptable salts of the compound of formula (I) are for example its formic acid salts.

The term "compound(s) of this invention" and "compound(s) of the present invention" refers to compounds of formula (I) and stereoisomers, tautomers, solvates, and salts (e.g., pharmaceutically acceptable salts) thereof.

Tautomeric forms, i.e. structural isomers which interconvert with the compound of formula (I), in particular in solution, may in some instances exist and are to be understood as being included in the invention.

If one of the starting materials or compounds of formula (I) contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compound of formula (I) can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

If desired, racemic mixtures of the compound of the invention may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where an optically pure enantiomer is provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer of the compound. A chirally pure or chirally enriched compound may be prepared by chirally selective synthesis or by separation of enantiomers. The separation of enantiomers may be carried out on the final product or alternatively on a suitable intermediate.

Structures depicted herein are also meant to include compounds that differ only in the presence of one or more isotopically enriched atoms. Particular examples of radioisotopes are ²H, ³H, ¹³C, ¹⁴C and ¹⁸F. For example the structures wherein one or more hydrogen atoms are replaced by deuterium or tritium, or one or more carbon atoms are replaced by a ¹³C- or ¹⁴C-enriched carbon are within the scope of this invention.

The invention thus relates to:
A compound according to the invention wherein R¹ is hydroxyalkyl or alkylcarbonyl;
A compound according to the invention wherein R¹ is hydroxyethyl or methylcarbonyl;
A compound according to the invention wherein R² is cyano, haloalkyl or haloalkoxy;
A compound according to the invention wherein R² is cyano, difluoromethyl or difluoromethoxy;
A compound according to the invention wherein R³ is hydrogen or methyl, and R⁴ is hydrogen; or R³ together with R⁴ and the atoms they are attached to, form a five- to seven-membered heterocycloalkyl or heteroaryl ring;
A compound according to the invention wherein R³ is hydrogen or methyl, and R⁴ is hydrogen; or R³ together with R⁴ and the atoms they are attached to, form 3,4-dihydro-2H-1,4-oxazine;
A compound according to the invention wherein R³ is hydrogen or alkyl, and R⁴ is hydrogen;
A compound according to the invention wherein R³ together with R⁴ and the atoms they are attached to, form a five- to seven-membered heterocycloalkyl or heteroaryl ring;
A compound according to the invention wherein R³ together with R⁴ and the atoms they are attached to, form 3,4-dihydro-2H-1,4-oxazine 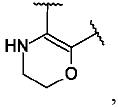 and wherein the double bond of 3,4-dihydro-2H-1,4-oxazine is part of the aromatic system of the pyridazine of the compound of formula (I);
A compound according to the invention wherein R⁵ is methyl or difluoromethyl;
A compound according to the invention wherein R⁵ is alkyl;
A compound according to the invention wherein R⁵ is methyl;
A compound according to the invention wherein A¹ is -N-; and
A compound according to the invention wherein A¹ is -CH-.

The invention further relates to a compound of formula (I) selected from
1-[3-acetyl-6-[6-[(6-methylpyridazin-3-yl)amino]imidazo[4,5-c]pyridin-3-yl]-2-pyridyl]-5-methyl-pyrazole-3-carbonitrile;
1-[3-(1-hydroxyethyl)-6-[6-[(6-methylpyridazin-3-yl)amino]imidazo[4,5-c]pyridin-3-yl]-2-pyridyl]-5-methyl-pyrazole-3-carbonitrile;
1-[3-(1-hydroxyethyl)-6-[6-[(6-methyl-3-pyridyl)amino]imidazo[4,5-c]pyridin-3-yl]-2-pyridyl]-5-methyl-pyrazole-3-carbonitrile;
1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanol;
1-[3-acetyl-6-[6-[methyl-(6-methylpyridazin-3-yl)amino]imidazo[4,5-c]pyridin-3-yl]-2-pyridyl]-5-methyl-pyrazole-3-carbonitrile;
1-[3-acetyl-6-[6-(3-methyl-6,7-dihydropyridazino[4,3-b][1,4]oxazin-8-yl)imidazo[4,5-c]pyridin-3-yl]-2-pyridyl]-5-methyl-pyrazole-3-carbonitrile; and
1-[2-[3-(difluoromethyl)-5-methyl-pyrazol-1-yl]-6-[6-[(6-methylpyridazin-3-yl)amino]imidazo[4,5-c]pyridin-3-yl]-3-pyridyl]ethanol;
or a pharmaceutically acceptable salt thereof.

The invention further relates to a compound of formula (I) selected from
1-[3-acetyl-6-[6-[(6-methylpyridazin-3-yl)amino]imidazo[4,5-c]pyridin-3-yl]-2-pyridyl]-5-methyl-pyrazole-3-carbonitrile;
1-[3-(1-hydroxyethyl)-6-[6-[(6-methylpyridazin-3-yl)amino]imidazo[4,5-c]pyridin-3-yl]-2-pyridyl]-5-methyl-pyrazole-3-carbonitrile; and
1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanol;
or a pharmaceutically acceptable salt thereof.

The synthesis of the compound of formula (I) can, for example, be accomplished according to the non-exhaustive procedures described below in general schemes 1-6 or according to methods known to those skilled in the art. In some instances, the sequence of the reaction steps can be modified and the individual steps of the different schemes can be combined in different ways as disclosed herein.

### Scheme 1

In scheme 1, the synthesis of a compound of formula (I-b) starting from a compound of formula (I-a) is described. In the scheme below A¹, R², R³, R⁴ and R⁵ are as defined above. The compound of formula (I-a) is a compound of formula (I) as described herein, wherein R¹ is methylcarbonyl. The compound of formula (I-b) is a compound of formula (I) as described herein, wherein R¹ is hydroxyethyl.

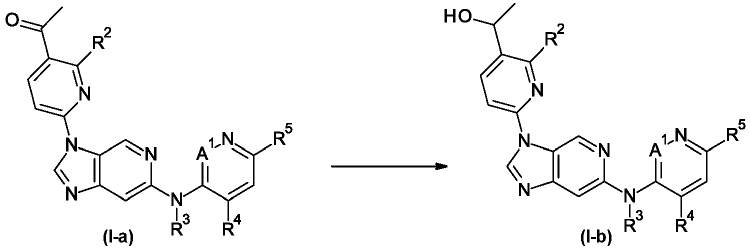

***Step A:*** A compound of formula **(I-a)** can be converted to a compound of formula **(I-b)** with a reducing agent such as NaBH₄ in MeOH with a cosolvent such as THF, 1,4-dioxane, EtOH, MeOH, DCM at a temperature between around -40 °C and RT.

### Scheme 2

In the scheme below A¹, R², R³, R⁴ and R⁵ are as defined above. The compound of formula (I-a) is a compound of formula (I) as described herein, wherein R¹ is methylcarbonyl.

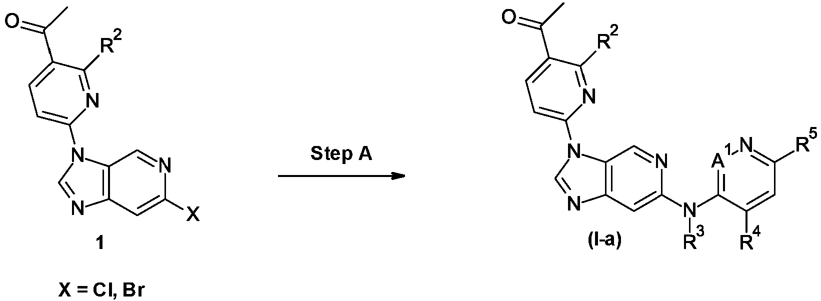

***Step A**:* Intermediate 1 can be converted to a compound of formula **(I-a)** via a Buchwald-Hartwig coupling of intermediate 3 with an aminopyridazine reactant using a suitable base such as for instance Cs₂CO₃, K₂CO₃ or K₃PO₄ and a suitable palladium catalyst such as for instance t-BuXPhos-Pd-G3 or [tBuBrettPhos Pd(allyl)]OTf in a suitable solvent such as 1,4-dioxane, optionally with water as cosolvent, at temperatures between around 80 °C and around 100°C.

### Scheme 3

In the scheme below R² is as described herein.

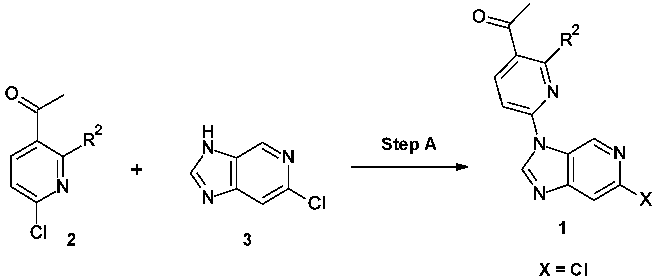

***Step A:*** Intermediate **2** and **3** can be reacted to give an intermediate **1** in the presence of a suitable base such as for instance DBU, K₂CO₃ or Cs₂CO₃ in a suitable solvent such as for instance 1,4-dioxane or DMSO at a temperature of around 80 °C.

### Scheme 4

In the scheme below A¹, R², R³, R⁴ and R⁵ are as defined above. The compound of formula (I-a) is a compound of formula (I) as described herein, wherein R¹ is methylcarbonyl.

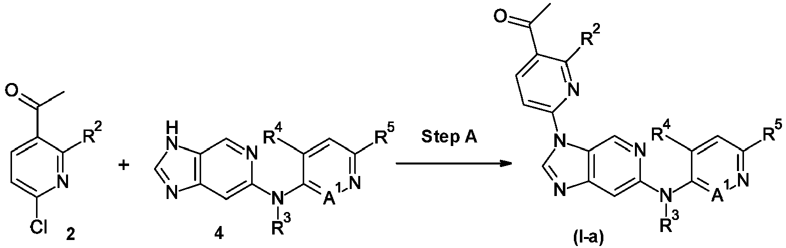

***Step A:*** Intermediate **2** and **4** can be reacted to give the compound of formula (I-a) in the presence of a suitable base such as for example DBU, K₂CO₃ or Cs₂CO₃ in a suitable solvent such as for example 1,4-dioxane, DMF, NMP, DMA or DMSO, optionally with water as cosolvent, at temperatures between around 50 °C and around 80 °C.

### Scheme 5

In the scheme below R² is as described herein.

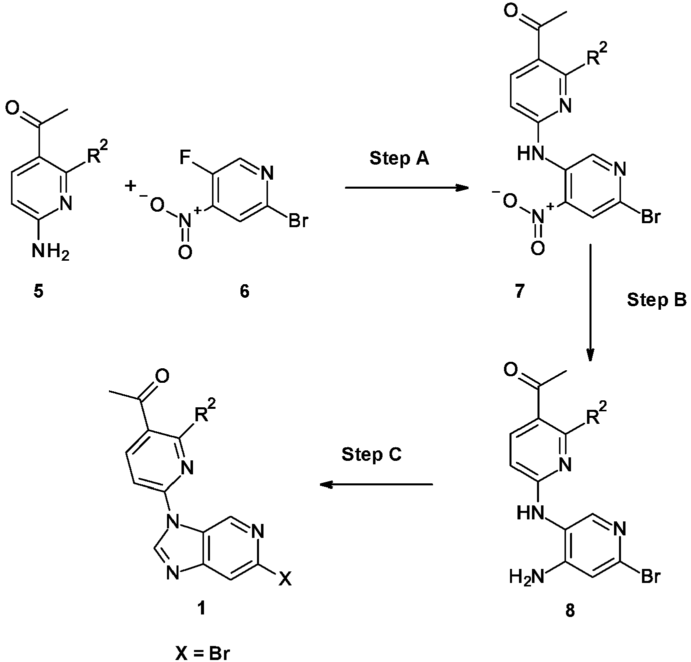

*Step A:* An intermediate **7** can be obtained by reacting an aniline **5** and a fluoro or chloropyridine **6** in a solvent such as DMF, NMP or DMA in the presence of a deprotonating agent such as for instance NaH at temperatures between around -10 °C and RT.

*Step B:* An aniline intermediate **8** can be obtained by reduction of the corresponding nitro precursor 7 using a reducing agent such as activated Zn in a solvent such as EtOH or water in the presence of HOAc at a temperature of around 50 °C.

*Step C:* An intermediate **1** can be obtained for aniline intermediate **8** by condensation with trimethoxymethane, optionally in the presence of TsOH, in a solvent such as MeOH at temperatures between around 80 °C and around 120 °C.

### Scheme 6

In scheme 6, the synthesis of intermediate 5a is described. The compound of formula 5a is a compound of formula 5, wherein R² is methyl-pyrazolyl optionally substituted with substituent R^{a} selected from carbonitrile, alkyl or haloalkyl, or substituent R^{b} selected from hydrogen, alkyl and haloalkyl.

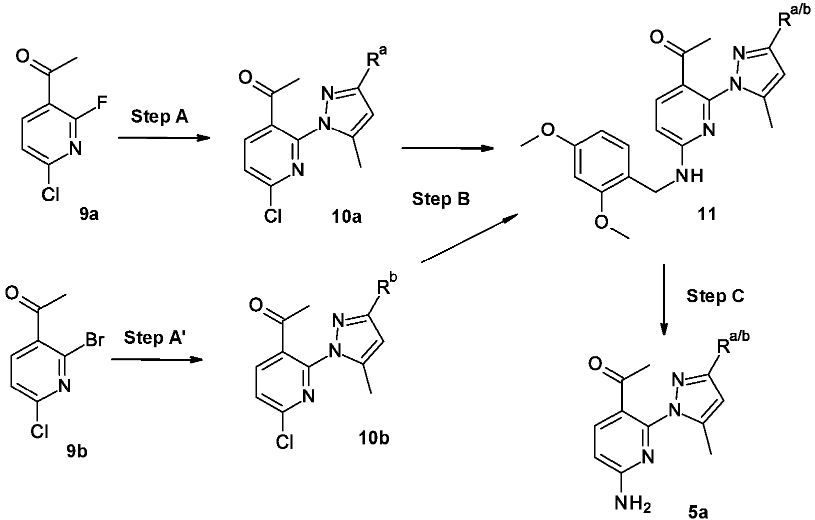

*Step A:* An intermediate **10a** can be obtained by reacting building block **9a** with a suitable pyrazole building block in a suitable solvent such as 1,4-dioxane, THF, DMF, DMA, NMP or DMSO in the presence of a suitable base such as DBU at a temperature of around 80 °C.

*Step A':* Intermediate **10b** can be obtained by reacting starting material **9b** with a suitable pyrazole boronic ester in the presence of a suitable base such as K₂CO₃ and a suitable catalyst such as Pd(dppf)Cl₂·CH₂Cl₂ in a solvent such as 1,4-dioxane, optionally with water as cosolvent, at a temperature of around 80 °C.

*Step B:* An intermediate **11** can be obtained by reacting an intermediate **10** with (2,4-dimethoxybenzyl)amine in a suitable solvent such as for example 1,4-dioxane, THF, DMF, DMA, NMP or DMSO in the presence of a suitable base such as for example DIPEA at temperatures between RT and around 80 °C.

*Step C:* An intermediate **5a** can be obtained by deprotecting an intermediate **11** with a strong acid such as for instance TFA in a suitable solvent such as for example DCM at RT.

The invention thus also relates to a process for the preparation of a compound according to the invention, comprising one of the following steps:
(a) the reaction of a compound of formula (B1) 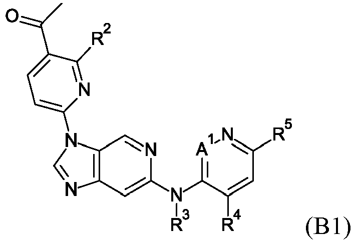 with a suitable reducing agent in presence of a suitable solvent,
(b) the reaction of a compound of formula (B2) 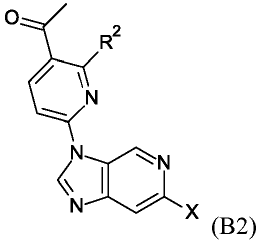 with a compound of formula (B3) 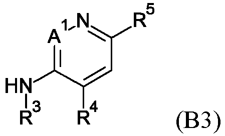 in presence of a suitable base, a suitable catalyst and a suitable solvent, or
(c) the reaction of a compound of formula (B4) 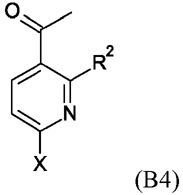 with a compound of formula (B5) 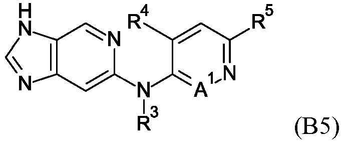 in presence of a suitable base.

In the above process, X is halogen; A¹, R², R³, R⁴ and R⁵ are as described herein.

In the reaction of step (a) the reducing agent can be for example LiBH₄ or NaBH₄.

In the reaction of step (a) the solvent can be for example MeOH optionally with a suitable cosolvent. The cosolvent can be for example THF, dioxane, EtOH, dichloromethane or a mixture thereof.

Convenient conditions for step (a) are between around -60 °C and around room temperature, in particular between around -40 °C and room temperature.

Conveniently, the conditions for step (a) are NaBH₄ as reducing agent, MeOH as solvent with a suitable cosolvent and a reaction temperature of between around -40 °C and room temperature.

In the reaction of step (b) the base can be for example Cs₂CO₃, K₂CO₃ or K₃PO₄.

In the reaction of step (b) the catalyst can be for example a suitable Pd catalyst, in particular t-BuXPhos-Pd-G3 or [tBuBrettPhos Pd(allyl)]OTf.

In the reaction of step (b) the solvent can be for example 1,4-dioxane, THF, DMA, DMF, NMP, toluene, xylene, water or a mixture thereof.

Convenient conditions for step (b) can be between around 60 °C and 120 °C, in particular around 70 °C and around 110 °C, more particular between around 80 °C and around 100 °C.

Conveniently, the conditions for step (b) are Cs₂CO₃ or K₂CO₃ as base, t-BuXPhos-Pd-G3 or [tBuBrettPhos Pd(allyl)]OTf as catalyst and 1,4-dioxane as solvent at a reaction temperature of between around 80 °C and around 100 °C.

In the reaction of step (c) the base can be for example DBU, K₂CO₃ or Cs₂CO₃.

In the reaction of step (c) the solvent can be for example 1,4-dioxane, DMF, NMP, DMA or DMSO.

Convenient conditions for step (c) can be between around 30 °C and 120 °C, in particular around 40 °C and around 100 °C, more particular between around 50 °C and around 80 °C.

Conveniently, the conditions for step (c) are Cs₂CO₃ or K₂CO₃ as base, t-BuXPhos-Pd-G3 or [tBuBrettPhos Pd(allyl)]OTf as catalyst and 1,4-dioxane as solvent at a reaction temperature of between around 80 °C and around 100 °C.

The invention also relates in particular to:
A compound of formula (I) as describe herein or a pharmaceutically acceptable salt thereof, for use as therapeutically active substance;
A pharmaceutical composition comprising a compound of formula (I) as described herein or a pharmaceutically acceptable salt thereof, and a therapeutically inert carrier;
A compound of formula (I) as described herein or a pharmaceutically acceptable salt thereof, for use in the treatment or prophylaxis of rheumatoid arthritis, juvenile rheumatoid arthritis, non-alcoholic steatohepatitis (NASH), primary sclerosing cholangitis, giant cell vasculitis, inflammatory bowel diseases (IBD), atherosclerosis, type 2 diabetes or glomerulonephritis, in particular inflammatory bowel diseases (IBD); and

### Pharmaceutical Compositions

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal, epidural and intranasal, and if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The invention will now be illustrated by the following examples.

### Examples

### Abbreviations

- [tBuBrettPhos Pd(allyl)]OTf: allyl(2-di-tert-butylphosphino-3,6-dimethoxy-2',4',6'-triisopropyl-1,1'-biphenyl)palladium(II) triflate (CAS # 1798782-15-6)
- ATP: adenosine triphosphate
- aq.: aqueous
- BINAP: (2,2'-bis(diphenylphosphino)-1,1'-binaphthyl)
- Boc: tert-butyloxycarbonyl
- DBU: 1,8-diazabicyclo[5.4.0]undec-7-ene
- DIPEA: N,N-diisopropylethylamine
- DMF: *N,N* -dimethylformamide
- DMSO: dimethyl sulfoxide
- dppf: 1,1'-ferrocenediyl-bis(diphenylphosphine)
- dtbpy: 4,4'-Di-tert-butyl-2,2'-dipyridyl
- equiv.: equivalents
- ESI: electrospray ionization
- Et: ethyl
- EtOAc: ethyl acetate
- EtOH: ethanol
- FA: formic acid
- HPLC: high pressure liquid chromatography
- LC-MS: high-performance liquid chromatography
- Me: methyl
- MeOH: methanol
- Ms: methanesulfonyl
- NMR: nuclear magnetic resonance
- PE: petroleum ether
- psi: pounds per square inch
- RT: room temperature
- sat.: saturated
- SFC: supercritical fluid chromatography
- sol.: solution
- TBD: triazabicyclodecene
- TBDMS: tert-butyldimethylsilyl
- [tBuBrettPhos Pd(allyl)]OTf: trifluoromethanesulfonate allyl[(2-di-tert-butylphosphino-3,6-dimethoxy-2',4',6'-triisopropyl-1,1'-biphenyl)-2-(2'-amino-1,1'-biphenyl)] palladium(II) (CAS # 1798782-15-6)
- t-BuXPhos-Pd-G3: [(2-Di-tert-butylphosphino-2',4',6'-triisopropyl-1,1'-biphenyl)-2-(2'-amino-1,1'-biphenyl)] palladium(II) methanesulfonate (CAS # 1447963-75-8)
- TEA: triethylamine
- Tf: triflyl
- TFA: trifluoroacetic acid
- TFAA: trifluoroacetic anhydride
- THF: tetrahydrofuran

### Example 1

### 1-[3-Acetyl-6-[6-[(6-methylpyridazin-3-yl)amino]imidazo[4,5-c]pyridin-3-yl]-2-pyridyl]-5-methyl-pyrazole-3-carbonitrile

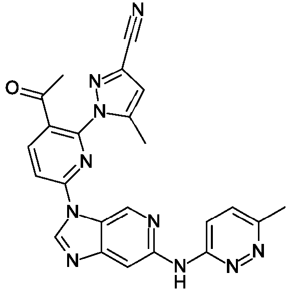

### Step 1: 1-[3-Acetyl-6-(6-chloroimidazo[4,5-c]pyridin-3-yl)-2-pyridyl]-5-methyl-pyrazole-3-carbonitrile

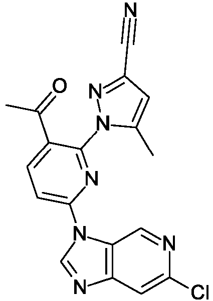

Under an argon atmosphere, 6-chloro-3H-imidazo[4,5-c]pyridine (CAS 2589-11-9, 100 mg, 0.65 mmol, 1 equiv.) was dissolved in dimethyl sulfoxide (5 mL). Then, 1-(3-acetyl-6-chloro-2-pyridyl)-5-methyl-pyrazole-3-carbonitrile (ex. 3, step 1; 254 mg, 0.98 mmol, 1.5 equiv.) and 1,8-diazabicyclo[5.4.0]undec-7-ene (CAS 6674-22-2; 109.0 mg, 108 uL, 0.72 mmol, 1.1 equiv.) were added. The reaction mixture was stirred at RT overnight. More 1,8-diazabicyclo[5.4.0]undec-7-ene (109 mg, 108 uL, 0.72 mmol, 1.1 equiv.) was added. The reaction mixture was stirred at 80 °C for 1 hour, then cooled to RT. It was partitioned between water and EtOAc and extracted. The organic layer was dried over MgSO₄, filtered and concentrated in vacuo. The crude product was purified by reversed phase chromatography using water/acetonitrile as eluent to provide a mixture of the two regioisomers (219 mg): The regioisomers were separated by SFC chromatography using 35% MeOH to provide the title compound (39 mg, 16%) as brown solid. LC-MS: m/z = 378.1 [M+H]⁺, ESI pos.

### Step 2: 1-[3-Acetyl-6-[6-[(6-methylpyridazin-3-yl)amino]imidazo[4,5-c]pyridin-3-yl]-2-pyridyl]-5-methyl-pyrazole-3-carbonitrile, formic acid

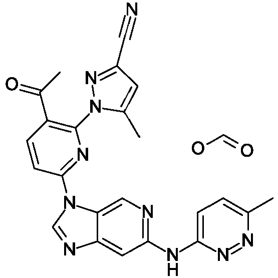

In a sealed tube, 1-[3-acetyl-6-(6-chloroimidazo[4,5-c]pyridin-3-yl)-2-pyridyl]-5-methylpyrazole-3-carbonitrile (41 mg, 0.11 mmol, 1 equiv.) was dissolved in 1,4-dioxane (4 mL). (6-methylpyridazin-3-yl)amine (23.7 mg, 0.22 mmol, 2 equiv.), Cs₂CO₃ (70.7 mg, 0.22 mmol, 2 equiv.) and water (29.3 mg, 29.3 uL, 1.63 mmol, 15 equiv.) were added. The reaction mixture was flushed with argon, then t-BuXPhos-Pd-G3 (17.2 mg, 0.022 mmol, 0.2 equiv.) was added. The reaction mixture was stirred at 90 °C. After 2.5 hours, t-BuXPhos-Pd-G3 (17.24 mg, 0.022 mmol, 0.20 equiv.) was added again, and stirring was continued for 1 hour at 90 °C. The reaction mixture was cooled to RT. One spatula of metal scavenger SiliaMetS^{®} Thiol was added. The reaction mixture was stirred at RT over 30 minutes and filtered, washing the residue on the filter with MeOH/DCM/FA (90/10/0.05). The filtrate was concentrated. The crude product was purified by silica gel chromatography using an DCM/MeOH gradient as eluent and then a reverse phase chromatograpy using a gradient of 1% aqueous formic acid/acetontrile as eluent to obtain the title compound (4.5 mg, 8%) as light yellow lyophilized powder. LC-MS: m/z = 453.3 [M+H]⁺, ESI pos.

### Example 2

### 1-[3-(1-Hydroxyethyl)-6-[6-[(6-methylpyridazin-3-yl)amino]imidazo[4,5-c]pyridin-3-yl]-2-pyridyl]-5-methyl-pyrazole-3-carbonitrile

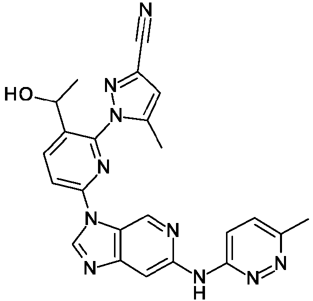

Under an argon atmosphere 1-[3-acetyl-6-[6-[(6-methylpyridazin-3-yl)amino]imidazo[4,5-c]pyridin-3-yl]-2-pyridyl]-5-methyl-pyrazole-3-carbonitrile (example 1; 234 mg, 0.14 mmol, 1 equiv.) was dissolved in methanol (3.5 mL). NaBH₄ (15.9 mg, 0.42 mmol, 3 equiv.) was added and the reaction mixture was stirred at RT over 30 minutes. The reaction mixture was diluted with water. The crude product was purified by reversed phase chromatography using a 0.1% aqueous trimethylamine/acetonitrile gradient as eluent to provide the title compound (15 mg, 23%) as lyophilized powder. LC-MS: m/z = 453.3 [M+H]⁺, ESI pos.

### Example 3

### 1-[3-(1-Hydroxyethyl)-6-[6-[(6-methyl-3-pyridyl)amino]imidazo[4,5-c]pyridin-3-yl]-2-pyridyl]-5-methyl-pyrazole-3-carbonitrile

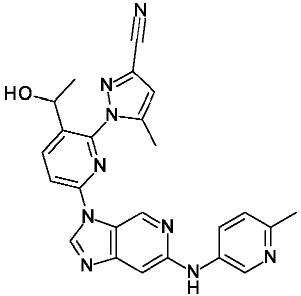

### Step 1: 1-(3-Acetyl-6-chloro-2-pyridyl)-5-methyl-pyrazole-3-carbonitrile

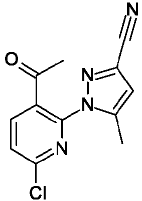

A solution of 1-(6-chloro-2-fluoro-3-pyridyl)ethanone (CAS# 1260663-13-5; 5 g, 28.81 mmol, 1 equiv.), 5-methyl-1*H*-pyrazole-3-carbonitrile (2.93 g, 27.4 mmol, 0.95 equiv.) and DIPEA (14.3 mL, 86.42 mmol, 3 equiv.) in DMSO (50 mL) was stirred at 80 °C for 4 hours. The reaction mixture was cooled to RT, poured into H₂O and extracted with EtOAc. The combined organic layers were washed with brine and concentrated. The crude product was purified by flash chromatography using a petroleum ether/EtOAc gradient as eluent to obtain the title compound (5.3 g, 71% yield) as yellow oil. LC-MS: m/z = 261.1 [M+H]⁺, ESI pos.

### Step 2: 1-[3-Acetyl-6-[(2,4-dimethoxybenzyl)amino]-2-pyridyl]-5-methyl-pyrazole-3-carbonitrile

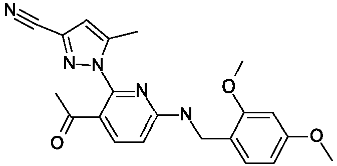

Under an argon atmosphere, 1-(3-acetyl-6-chloro-2-pyridyl)-5-methyl-pyrazole-3-carbonitrile (1.14 g, 4.37 mmol, 1 equiv.) was dissolved in DMSO (8.6 mL). DIPEA (1.9 mL, 10.9 mmol, 2.5 equiv.) and (2,4-dimethoxybenzyl)amine (803 mg, 723 uL, 4.8 mmol, 1.1 equiv.) were added. The reaction mixture was stirred at RT overnight. More (2,4-dimethoxybenzyl)amine (802.9 mg, 723 uL, 4.8 mmol, 1.1 equiv.) was added and the reaction mixture was again stirred at RT overnight. Then, it was partitioned between water and EtOAc. The organic layer was dried over MgSO₄; then filtered and concentrated in vacuo. The crude product was purified by silica gel chromatography using a heptane/ethyl acetate gradient as eluent to provide the title compound (1.85 g, quant.) as yellow oil. LC-MS: m/z = 392.2 [M+H]⁺, ESI pos.

### Step 3: 1-(3-Acetyl-6-amino-2-pyridyl)-5-methyl-pyrazole-3-carbonitrile

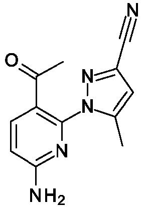

Under an argon atmosphere, 1-[3-acetyl-6-[(2,4-dimethoxybenzyl)amino]-2-pyridyl]-5-methylpyrazole-3-carbonitrile (1.95 g, 4.73 mmol, 1 equiv.) was dissolved in DCM, extra dry (14.25 mL). TFA (10.25 g, 6.93 mL, 89.92 mmol, 19 equiv.) was added, and the reaction mixture was stirred at RT over 30 minutes turning into a dark red solution. Then, it was neutralised with trietylamine and concentrated. The residue was tritured in MeOH overnight and then filtered. The filtrate was concentrated. The crude product was purified by reverse phase chromatography with NH₃ to give the title compound (830 mg, 72%) as light brown powder. LC-MS: m/z = 242.1 [M+H]⁺, ESI pos.

### Step 4: 2-Bromo-5-fluoro-1-oxido-pyridin-1-ium

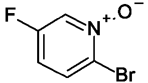

Under an argon atmosphere, 2-bromo-5-fluoro-pyridine (CAS 41404-58-4; 4.12 g, 22.9 mmol, 1 equiv.) was dissolved in DCM (41.2 mL). At 0 °C, TFAA (14.5 g, 9.7 mL, 68.8 mmol, 3 equiv.) was added, followed by the dropwise addition over 30 minutes of H₂O₂ (2.68 g, 2.4 mL, 27.5 mmol, 1.2 equiv.): very exothermic! The reaction mixture was stirred at RT overnight, then carefully poured into ice cold water. The aqueous layer was extracted several times with DCM. The combined organic layers were dried over MgSO₄ and concentrated in vacuo at RT. The compound started to solidify under heat formation and was therefore cooled in an ice bath for 30 minutes. The crude was used at the next step without any further purification.

Dynamic DSC scan (30-500 °C, 10 °C/min): onset temperature for exothermic decomposition 110 °C, enthalpy: 490 J/g.

### Step 5: 2-Bromo-5-fluoro-4-nitro-pyridine

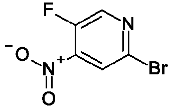

Under an argon atmosphere, 2-bromo-5-fluoro-1-oxido-pyridin-1-ium (4.4 g, 18.33 mmol, 1 equiv.) was dissolved in concentrated H₂SO₄ (28.2 g, 15.3 mL, 287 mmol, 15.6 equiv.) to give a pink solution. At 0 °C, fuming HNO₃ (10.8 g, 7.65 mL, 171.2 mmol, 9.3 equiv.) was added slowly (exotherm: < 10 °C), yielding a pale yellow solution. The reaction mixture was heated to 120 °C over 12 hours . The developing nitrogen oxides fumes were trapped in cooled aquous NaOH solution.

After cooling to RT, the reaction mixture was poured onto ice cold water, brought to pH 6 with 4M NaOH and extracted with DCM. The combined organic extracts were dried over MgSO₄ and concentrated. The crude product was purified by silica gel chromatography using a heptane/ethyl acetate gradient as eluent and with a second silica gel chromatography using DCM/MeOH gradient as eluent to provide the title compound (1.33 g, 33%) as yellow oil.

Dynamic DSC scan (30-400 °C, 10 °C/min): Tm = 148 °C: onset of temperature for exothermic decomposition: ca. 173 °C, enthalpy: 1594 J/g. Highly energetic destruction!

### Step 6: 1-[3-Acetyl-6-[(6-bromo-4-nitro-3-pyridyl)amino]-2-pyridyl]-5-methyl-pyrazole-3-carbonitrile

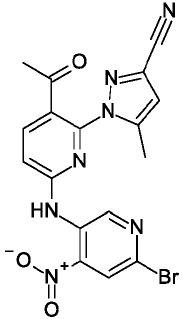

Under an argon atmosphere, 1-(3-acetyl-6-amino-2-pyridyl)-5-methyl-pyrazole-3-carbonitrile (650 mg, 2.7 mmol, 1 equiv.) was dissolved in DMF, extra dry (10.8 mL). At -10 °C, NaH (168.1 mg, 3.5 mmol, 1.3 equiv.) was added (gas evolution) followed by a solution containing 2-bromo-5-fluoro-4-nitro-pyridine (654.9 mg, 2.96 mmol, 1.1 equiv.) in DMF, extra dry (5.4 mL). The reaction mixture turned black and was stirred at -10 °C over 30 minutes. A second portion of 2-bromo-5-fluoro-4-nitro-pyridine (178.6 mg, 0.81 mmol, 0.3 equiv.) was added, and the reaction mixture was stirred at -10 °C over 15 minutes. Then, it was partitioned between NH₄Cl solution and EtOAc. The organic layer was dried over MgSO₄, filtered; concentrated in vacuo. The crude product was purified by silica gel chromatography using a DCM/MeOH gradient as eluent to provide the title compound (520 mg, 43%) as orange gum. LC-MS: m/z = 442.1 [M+H]⁺, ESI pos.

### Step 7: 1-[3-Acetyl-6-[(4-amino-6-bromo-3-pyridyljamino]-2-pyridyl]-5-methyl-pyrazole-3-carbonitrile

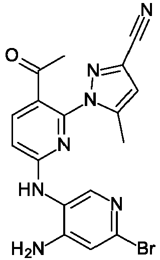

Under an argon atmosphere and in a flask equipped with a mechanic stirrer, 1-[3-acetyl-6-[(6-bromo-4-nitro-3-pyridyl)amino]-2-pyridyl]-5-methyl-pyrazole-3-carbonitrile (0.92 g, 2.1 mmol, 1 equiv.) was suspended in ethanol (15.3 mL). Activated zinc (1.36 g, 20.8 mmol, 10 equiv.) was added, and the orange suspension was cooled to 0 °C. A solution containing acetic acid (874 mg, 834 uL, 14.6 mmol, 7 equiv.) in ethanol (7.7 mL) was added dropwise while the temperature was kept under 5 °C. The reaction mixture became black and was stirred at 0 °C over 30 minutes, then for 1 hour at RT. The reaction mixture was filtered, and the cake was washed with EtOAc. The filtrate was concentrated to obtain the crude product which was used in the next step without further purification. LC-MS: m/z = 413.1 [M+H]+, ESI pos

### Step 8: 1-[3-Acetyl-6-(6-bromoimidazo[4,5-c]pyridin-3-yl)-2-pyridyl]-5-methyl-pyrazole-3-carbonitrile

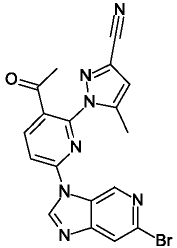

Under an argon atmosphere, 1-[3-acetyl-6-[(4-amino-6-bromo-3-pyridyl)amino]-2-pyridyl]-5-methyl-pyrazole-3-carbonitrile (0.86 g, 2.1 mmol, 1 equiv.) was dissolved in trimethoxymethane (14.47 g, 14.9 mL, 136.4 mmol, 65.5 equiv.). The reaction mixture was stirred at 90 °C for 4 hours. The volatiles was evaporated. The crude product was purified by chromatography on silica gel using a DCM/MeOH gradient as eluent and a second chromatography on silica gel using a heptane/ethyl acetate gradient as eluent to provide the title compound (113 mg, 13 %) as red solid. LC-MS: m/z = 424.1 [M+H]⁺, ESI pos.

### Step 9: 1-[3-Acetyl-6-[6-[(6-methyl-3-pyridyl)amino]imidazo[4,5-c]pyridin-3-yl]-2-pyridyl]-5-methyl-pyrazole-3-carbonitrile

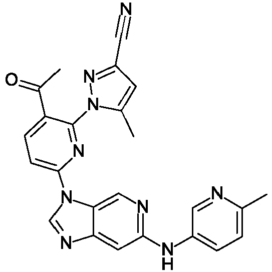

In analogy to the procedure described in example 1, step 2, using (6-methyl-3-pyridyl)amine as amine component, the title compound was obtained as yellow oil. LC-MS: m/z = 450.3 [M+H]⁺, ESI pos.

### Step 10: 1-[3-(1-Hydroxyethyl)-6-[6-[(6-methyl-3-pyridyl)amino]imidazo[4,5-c]pyridin-3-yl]-2-pyridyl]-5-methyl-pyrazole-3-carbonitrile, formic acid

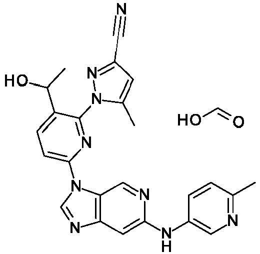

Under an argon atmosphere, 1-[3-acetyl-6-[6-[(6-methyl-3-pyridyl)amino]imidazo[4,5-c]pyridin-3-yl]-2-pyridyl]-5-methyl-pyrazole-3-carbonitrile (3 mg, 0.007 mmol, 1 equiv.) was dissolved in MeOH (0.165 mL). NaBH₄ (757 ug, 0.020 mmol, 3 equiv.) was added. The reaction mixture was stirred at RT over 30 minutes, then quenched by the addition of water. The reaction mixture was purified by reversed phase chromatography using 0.1% aqueous formic acid/acetonitrile as euluent to provide the title compound (1 mg, 29%) as white lyophilized powder. LC-MS: m/z = 496.4 [M-H]⁻, ESI neg.

### Example 4

### 1-[6-[6-[(6-Methylpyridazin-3-yl)amino]imidazo[4,5-c]pyridin-3-yl]-2-[3-methyl-1-(2,2,2-trifluoroethyl)pyrazol-4-yl]-3-pyridyl]ethanol

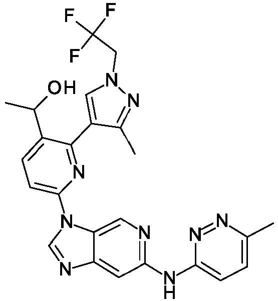

### Step 1: 4-Bromo-3-methyl-1-(2,2,2-trifluoroethyl)pyrazole

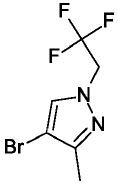

A mixture of 4-bromo-3-methylpyrazole (3:1 mixture with regioisomer; 15.0 g, 93.2 mmol, 1.0 equiv), 2,2,2-trifluoroethyl trifluoromethanesulfonate (22.71 g, 97.8 mmol, 1.05 equiv.) and CS₂CO₃ (25.33 g, 186.3 mmol, 2.0 equiv.) in DMF (150 mL) was stirred at 100 °C for 12 hours. Then, the reaction mixture was filtered. The filtrate was diluted with H₂O and extracted with ethyl acetate. The combined organic layers were were washed with saturated aqueous NaCl solution, dried over anhydrous Na₂SO₄, filtered and concentrated. The crude product was purified by silica gel chromatography to give a 2:1-mixture of the title compound and its regioisomer 4-bromo-3-methyl-1-(2,2,2-trifluoroethyl)pyrazole (19.5 g, 80.2 mmol, 86%) as off-white oil LC-MS: m/z = 242.9 [M+H]⁺, ESI pos.

¹H NMR (400 MHz, DMSO-d₆) δ = 8.00 (s, 1H), 5.09 - 4.99 (m, 2H), 2.14 (s, 3H).

### Step 2: 3-Methyl-4-(4,4,5,5-tetramethyl-1,3,2-dioxaborolan-2-yl)-1-(2,2, 2-trifluoroethyl)pyrazole

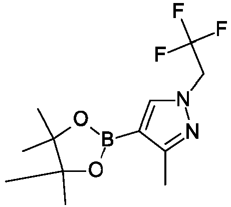

To a mixture of bis(pinacolato)diboron (17.24 g, 67.9 mmol, 1.5 equiv.) and a 2:1 mixture of 4-bromo-3-methyl-1-(2,2,2-trifluoroethyl)pyrazole and 4-bromo-5-methyl-1-(2,2,2-trifluoroethyl)pyrazole (11.0 g, 45.26 mmol, 1.0 equiv.) in 1,4-dioxane (200 mL) was added Pd(dppf)Cl₂·CH₂Cl₂ (3.7 g, 4.53 mmol, 0.1 equiv.) and potassium acetate (5.66 mL, 90.53 mmol, 2.0 equiv.). The reaction mixture was stirred at 100 °C for 16 hours under an nitrogen atmosphere, then concentrated. The crude product was purified by flash chromatography on silica gel using a petroleum ether/ethyl acetate gradient as eluent (0% - 20%) and and by a second chromatography on by preparative HPLC using a hexanes/ethyl acetate gradient as eluent to give a first crop of the title compound (3:1 mixture of isomers; 6.0 g, 20.7 mmol, 33% yield) as off-white oil and a second crop of the title compound (2:1 mixture of isomers (2.0 g, 6.9 mmol, 11%) as yellow oil. LC-MS: m/z = 291.1 [M+H]⁺, ESI pos.

### Step 3: 1-[6-Chloro-2-[3-methyl-1-(2,2,2-trifluoroethyl)pyrazol-4-yl]-3-pyridyl]ethanone

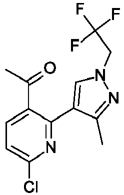

A mixture of 3-methyl-4-(4,4,5,5-tetramethyl-1,3,2-dioxaborolan-2-yl)-1-(2,2,2-trifluoroethyl)-pyrazole (1.0 g, 3.45 mmol, 1.0 equiv.), 1-(2-bromo-6-chloro-3-pyridyl)ethanone (808 mg, 3.45 mmol, 1.0 eq), potassium carbonate (953 mg, 6.89 mmol, 2.0 eq) and Pd(dppf)Cl₂·CH₂Cl₂ (281 mg, 0.34 mmol, 0.1 equiv.) in 1,4-dioxane (20 mL) and water (2 mL) was stirred at 80 °C under a nitrogen atmosphere for 12 hours. The reaction mixture was concentrated. The crude product was purified by column on silica gel using as petroleum ether/ethyl acetate gradient as eluent to provide the title compound (790.0 mg, 2.29 mmol, 72% yield) as yellow oil. LC-MS: m/z = 318.1, [M+H]⁺, ESI pos.

### Step 4a: N-(6-Methylpyridazin-3-yl)-3-(2-trimethylsilylethoxymethyl)imidazo[4,5-c]pyridin-6-amine

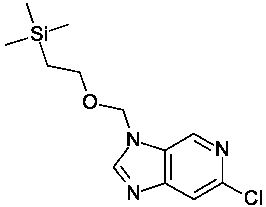

To a solution of 6-chloro-3H-imidazo[4,5-c]pyridine (880.0 mg, 5.73 mmol, 1.0 equiv.) in DMF (10 mL) was added 2-(trimethylsilyl)ethoxymethyl chloride (2.03 mL, 11.5 mmol, 2.0 equiv.) and TEA (1.59 mL, 11.46 mmol, 2.0 equiv.). The mixture was stirred at 20 °C for 2 hours. Then, water was added, followed by extraction with ethyl acetate. The combined organics were washed with NaCl solution, then concentrated under reduced pressure to give a residue. The crude product was purified by column on silica using a petroleum ether/ethyl acetate gradient as eluent to provide the title compound (1.1 g, 54%) as yellow oil. LC-MS: m/z = 284.0, [M+H]⁺, ESI pos.

### Step 5: N-(6-Methylpyridazin-3-yl)-3-(2-trimethylsilylethoxymethyl)imidazo[4,5-c]pyridin-6-amine

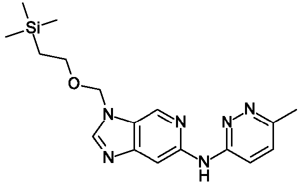

A mixture of 2-[(6-chloroimidazo[4,5-c]pyridin-3-yl)methoxy]ethyl-trimethyl-silane (900.0 mg, 3.17 mmol, 1.0 equiv.), 3-amino-6-methylpyridazine (1.04 g, 9.51 mmol, 3.0 eq), Cs₂CO₃ (2.06 g, 6.3 mmol, 2.0 equiv.) and allyl(trifluoromethylsulfonyloxy)palladiumditert-butyl-[3,6-dimethoxy-2-(2,4,6-triisopropylphenyl)phenyl]phosphane (248 mg, 0.32 mmol, 0.1 equiv.) in 1,4-dioxane (30 mL) was stirred at 100 °C under a nitrogen atmosphere for 12 hours. The mixture was filtered and concentrated. The crude product was purified by reversed phase flash using a acetonitrile/0.1% aqueous formic acid gradient as eluent to give the title compound (670.0 mg, 1.88 mmol, 59% yield) as a yellow solid. LC-MS: m/z = 357.0, [M+H]⁺, ESI pos.

### Step 5: N-(6-Methylpyridazin-3-yl)-3H-imidazo[4,5-c]pyridin-6-amine

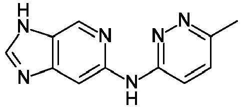

A solution of N-(6-methylpyridazin-3-yl)-3-(2-trimethylsilylethoxymethyl)imidazo[4,5-c]pyridin-6-amine (670 mg, 1.88 mmol, 1.0 equiv.) in TFA (7.0 mL) was stirred at 25 °C for 2 hours. The mixture was concentrated. The crude product was purified by reversed phase flash using a acetonitrile/ 0.1% aqueous formic acid gradient as eluent to give the title compound (580 mg, 2.56 mmol, 95% yield) as a yellow solid. LC-MS: m/z = 227.2, [M+H]⁺, ESI pos.

¹H NMR (400 MHz, DMSO-d₆) δ = 11.59 - 11.31 (m, 1H), 8.89 (s, 1H), 8.55 (s, 1H), 7.84 - 7.74 (m, 3H), 2.57 (s, 3H).

### Step 6: 1-[6-[6-[(6-Methylpyridazin-3-yl)amino]imidazo[4,5-c]pyridin-3-yl]-2-[3-methyl-1-(2,2,2-trifluoroethyl)pyrazol-4-yl]-3-pyridyl]ethanone

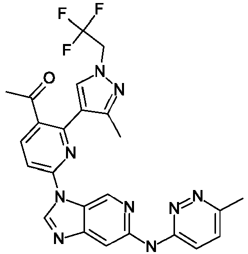

To a solution of N-(6-methylpyridazin-3-yl)-3H-imidazo[4,5-c]pyridin-6-amine (200.0 mg, 0.62 mmol, 1.0 equiv.) in DMSO (5 mL) was added 1-[6-chloro-2-[3-methyl-1-(2,2,2-trifluoroethyl)pyrazol-4-yl]-3-pyridyl]ethanone (197 mg, 0.62 mmol, 1.0 eq) and potassium carbonate (171 mg, 1.24 mmol, 2.0 eq). The mixture was stirred at 70 °C for 12 hours, then quenched with water and filtrated. The filtrate was extracted with ethyl acetate. The organic layer was washed with brine, dried over with anhydrous sodium sulfate and concentrated. The residue was purified by reversed phase flash using a acetonitrile/0.1% aqueous formic acid gradient to provide a mixture of 1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanone, 1-[6-[6-[(6-methyl-pyridazin-3-yl)amino]imidazo[4,5-c]pyridin-3-yl]-2-[5-methyl-1-(2,2,2-trifluoroethyl)pyrazol-4-yl]-3-pyridyl]ethanone, 1-[6-[6-[(6-methyl-pyridazin-3-yl)amino]imidazo[4,5-c]pyridin-1-yl]-2-[3-methyl-1-(2,2,2-trifluoroethyl)pyrazol-4-yl]-3-pyridyl]ethanone and 1-[6-[6-[(6-methyl-pyridazin-3-yl)amino]imidazo[4,5-c]pyridin-1-yl]-2-[5-methyl-1-(2,2,2-trifluoroethyl)pyrazol-4-yl]-3-pyridyl]ethanone (150.0 mg, 0.3 mmol, 48% yield) as yellow solid. LC-MS: m/z = 508.1, [M+H]⁺, ESI pos.

The mixture of the 4 isomers was purified by SFC (0.1% NH₃H₂O in isopropyl alcohol) to give two mixtures of two isomers each.

1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanone and 1-[6-[6-[(6-methylpyridazin-3-yl)amino]imidazo[4,5-c]pyridin-3-yl]-2-[5-methyl-1-(2,2,2-trifluoroethyl)pyrazol-4-yl]-3-pyridyl]ethanone (70 mg).

1-[6-[6-[(6-methylpyridazin-3-yl)amino]imidazo[4, 5-c]pyridin-1-yl]-2-[3-methyl-1-(2,2,2-trifluoroethyl)pyrazol-4-yl]-3-pyridyl]ethanone and 1-[6-[6-[(6-methylpyridazin-3-yl)amino]imidazo[4,5-c]pyridin-1-yl]-2-[5-methyl-1-(2,2,2-trifluoroethyl)pyrazol-4-yl]-3-pyridyl]ethanone (50 mg).

LC-MS: m/z = 508.3 [M+H]⁺, ESI pos.

The mixture of 1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanone and 1-[6-[6-[(6-methylpyridazin-3-yl)amino]imidazo[4,5-c]pyridin-3-yl]-2-[5-methyl-1-(2,2,2-trifluoroethyl)pyrazol-4-yl]-3-pyridyl]ethanone (70.0 mg) was separated by SFC (0.1% NH₃H₂O in isopropyl alcohol) to give the title compound 1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanone (25 mg) as a yellow solid. LC-MS: m/z = 508.2, [M+H]⁺, ESI pos.
¹H NMR (400 MHz, DMSO-d₆) δ = 9.94 (s, 1H), 9.32 (d, J = 0.8 Hz, 1H), 9.29 (s, 1H), 8.36 (d, J = 0.6 Hz, 1H), 8.29 (d, J = 8.6 Hz, 1H), 8.09 (s, 1H), 8.07 (d, J = 8.6 Hz, 1H), 7.72 (d, J = 9.1 Hz, 1H), 7.40 (d, J = 9.1 Hz, 1H), 5.21 - 5.10 (m, 2H), 2.51 (br s, 3H), 2.36 (s, 3H), 2.30 (s, 3H)
   and
1-[6-[6-[(6-methylpyridazin-3-yl)amino]imidazo[4,5-c]pyridin-3-yl]-2-[5-methyl-1-(2,2,2-trifluoroethyl)pyrazol-4-yl]-3-pyridyl]ethanone (12 mg) as a white solid. LC-MS: m/z = 508.2, [M+H]⁺, ESI pos.
¹H NMR (400 MHz, DMSO-d₆) δ = 10.04 (s, 1H), 9.01 (s, 1H), 8.81 (s, 1H), 8.77 (s, 1H), 8.35 (d, J = 8.5 Hz, 1H), 7.95 (d, J = 8.5 Hz, 1H), 7.90 (s, 1H), 7.74 (d, J = 9.1 Hz, 1H), 7.41 (d, J = 9.1 Hz, 1H), 5.26 - 5.14 (m, 2H), 2.52 (br s, 3H), 2.41 (s, 3H), 2.41 (s, 3H)

The mixture of 1-[6-[6-[(6-methylpyridazin-3-yl)amino]imidazo[4,5-c]pyridin-1-yl]-2-[3-methyl-1-(2,2,2-trifluoroethyl)pyrazol-4-yl]-3-pyridyl]ethanone and 1-[6-[6-[(6-methylpyridazin-3-yl)amino]imidazo[4,5-c]pyridin-1-yl]-2-[5-methyl-1-(2,2,2-trifluoroethyl)pyrazol-4-yl]-3-pyridyl]ethanone (50 mg) was purified by SFC (0.1% NH₃H₂O in isopropyl alcohol) to give
1-[6-[6-[(6-methylpyridazin-3-yl)amino]imidazo[4,5-c]pyridin-1-yl]-2-[3-methyl-1-(2,2,2-trifluoroethyl)pyrazol-4-yl]-3-pyridyl]ethanone and (35 mg) as a white solid. LC-MS: m/z = 508.2, [M+H]⁺, ESI pos.
¹H NMR (400 MHz, DMSO-d₆) δ = 10.03 (s, 1H), 9.08 (s, 1H), 9.02 (s, 1H), 8.78 (s, 1H), 8.54 (s, 1H), 8.37 (d, J = 8.4 Hz, 1H), 7.97 (d, J = 8.5 Hz, 1H), 7.63 (d, J = 9.1 Hz, 1H), 7.45 (d, J = 9.1 Hz, 1H), 5.24 - 5.13 (m, 2H), 2.54 (s, 3H), 2.44 (s, 3H), 2.14 (s, 3H)
   and
1-[6-[6-[(6-methylpyridazin-3-yl)amino]imidazo[4,5-c]pyridin-1-yl]-2-[5-methyl-1-(2,2,2-trifluoroethyl)pyrazol-4-yl]-3-pyridyl]ethanone (18 mg) as a yellow solid. LC-MS: m/z = 508.2, [M+H]⁺, ESI pos.
¹H NMR (400 MHz, DMSO-d₆) δ = 9.94 (s, 1H), 9.33 (s, 1H), 9.31 (s, 1H), 8.38 (s, 1H), 8.34 (d, J = 8.5 Hz, 1H), 8.07 (d, J = 8.6 Hz, 1H), 7.75 (s, 1H), 7.70 (d, J = 9.1 Hz, 1H), 7.40 (d, J = 9.1 Hz, 1H), 5.28 - 5.18 (m, 2H), 2.53 - 2.51 (m, 3H), 2.41 (s, 3H), 2.40 (s, 3H).

### Step 7: 1-[6-[6-[(6-Methylpyridazin-3-yl)amino]imidazo[4,5-c]pyridin-3-yl]-2-[3-methyl-1-(2,2,2-trifluoroethyl)pyrazol-4-yl]-3-pyridyl]ethanol, formic acid

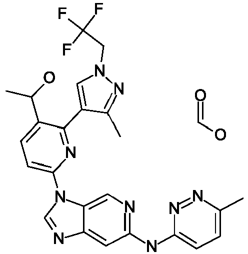

To a solution of 1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanone (23.0 mg, 0.05 mmol, 1.0 eq) in methanol (2 mL) was added NaBH₄ (8.57 mg, 0.23 mmol, 5.0 equiv.) at -70 °C. The mixture was stirred at -70 °C for 0.5 hour. The reaction mixture was quenched by addition of aqueous NH₄Cl at 20 °C. The crude product was purified by reversed phase HPLC using 0.1% aqueous formic acid /acetonitrile as eluent to provide the title compound (11.0 mg, 0.02 mmol, 46% yield) as a yellow solid. LC-MS: m/z = 510.3, [M+H]⁺, ESI pos.

¹H NMR (400 MHz, MeOH-d₄) δ = 9.32 (s, 1H), 9.07 (s, 1H), 8.29 (d, J = 8.5 Hz, 1H), 8.25 (s, 1H), 8.13 (s, 1H), 8.02 (s, 1H), 7.89 (d, J = 8.5 Hz, 1H), 7.75 (d, J = 9.3 Hz, 1H), 7.47 (d, J = 9.1 Hz, 1H), 5.04 - 4.95 (m, 3H), 2.56 (s, 3H), 2.35 (s, 3H), 1.46 (d, J = 6.4 Hz, 3H).

### Example 5

### 1-[3-acetyl-6-[6-[methyl-(6-methylpyridazin-3-yl)amino]imidazo[4,5-c]pyridin-3-yl]-2-pyridyl]-5-methyl-pyrazole-3-carbonitrile

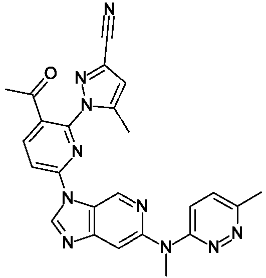

### Step 1: 1-[3-Acetyl-6-(6-chloroimidazo[4,5-c]pyridin-3-yl)-2-pyridyl]-5-methyl-pyrazole-3-carbonitrile (corresponding to Example 1, step 1)

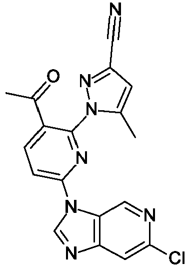

A solution of 6-chloro-3H-imidazo[4,5-c]pyridine (520 mg, 3.381 mmol, 1.0 equiv.), 1-(3-acetyl-6-chloro-2-pyridyl)-5-methyl-pyrazole-3-carbonitrile (971 mg, 3.7 mmol, 1.1 equiv.) in dimethyl sulfoxide (15.6 mL) with 1,8-diazabicyclo[5.4.0]undec-7-ene (567 mg, 561 uL, 3.72 mmol, 1.1 equiv.) was stirred at 80 °C for 3 hours. Again, 1,8-diazabicyclo[5.4.0]undec-7-ene (567 mg, 561 uL, 3.72 mmol, 1.1 equiv.) was added and stirring at 80 °C was continued for 6 hours. The reaction mixture was poured on water and extracted with EtOAc. The organic layers were dried over Na₂SO₄, filtered and concentrated. The crude product was purified by silica gel chromatography using a DCM/MeOH gradient as eluent to provide the title compound (456 mg, 36%) as light brown solid

### Step 2: 1-[3-acetyl-6-[6-[methyl-(6-methylpyridazin-3-yl)amino]imidazo[4,5-c]pyridin-3-yl]-2-pyridyl]-5-methyl-pyrazole-3-carbonitrile

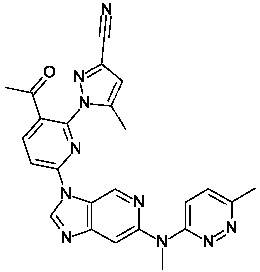

A suspension of 1-[3-acetyl-6-(6-chloroimidazo[4,5-c]pyridin-3-yl)-2-pyridyl]-5-methylpyrazole-3-carbonitrile (125 mg, 0.288 mmol, 1.0 equiv), methyl-(6-methylpyridazin-3-yl)amine (CAS 18591-82-7; 73.4 mg, 0.595 mmol, 2.0 equiv.) and Cs₂CO₃ (485.1 mg, 1.488 mmol, 5.0 equiv.) at RT in 1,4-dioxane (8.2 mL) was flushed with argon for 5 minutes. Then [tBuBrettPhos Pd(allyl)]OTf (23.3 mg, 0.03 mmol, 0.10 equiv. ) was added. The vial was sealed. The reaction mixture mixture was heated to 80 °C for 2 hours. Again [tBuBrettPhos Pd(allyl)]OTf (23.26 mg, 0.03 mmol, 0.10 equiv.) was added and stirring at 80 °C was continued for 2 hours. Then, the reaction mixture was concentrated. The crude product was purified by silica gel chromatography using a DCM/MeOH gradient and a second silica gel chromatography using a EtOAc/MeOH gradient as eluent to provide the title compound (33 mg, 30%) as white solid. LC-MS: m/z = 242.1 [M+H]⁺, ESI pos.

### Example 6

### 1-[3-Acetyl-6-[6-(3-methyl-6,7-dihydropyridazino[4,3-b][1,4]oxazin-8-yl)imidazo[4,5-c]pyridin-3-yl]-2-pyridyl]-5-methyl-pyrazole-3-carbonitrile

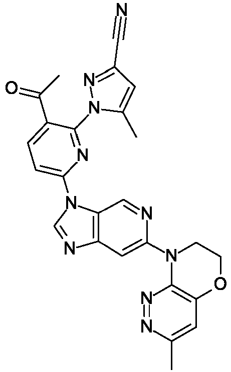

### Step 1: 3,6-Dichloro-4-(2-iodoethoxy)pyridazitie

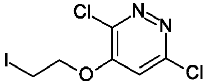

To the solution of 3,4,6-trichloropyridazine (4.5 g, 24.5 mmol, 1 equiv.) in THF (100 mL) was added 2-iodoethanol (4.43 g, 25.8 mmol, 1.05 equiv.) at 0 °C under an nitrogen atmosphere. The reaction mixture was stirred at 0 °C for 30 minutes. Then, NaH (1.18 g, 29.44 mmol, 1.2 eq) was added at 0 °C. The reaction mixture was stirred at 50 °C for 16 hours, then taken up in saturated aqueous NH₄Cl solution and extracted with EtOAc. The combined organic layer was dried over Na₂SO₄, filtered and concentrated. The crude product was purified by silica gel chromatography using a petroleum ether/ethyl acetate gradient as eluent to provide the title compound (3 g, 9.41 mmol, 38% yield) as light yellow solid. LC-MS: m/z = 318.9 [M+H]⁺, ESI pos.

### Step 2: 2-(3,6-Dichloropyridazin-4-yl)oxy-N-[(2,4-dimethoxyphenyl)methyl]ethanamine

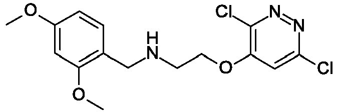

To a solution of 3,6-dichloro-4-(2-iodoethoxy)pyridazine (3.0 g, 9.41 mmol, 1 equiv.) and 2,4-dimethoxybenzylamine (1.7 mL, 11.3 mmol, 1.2 equiv.) in DMSO (30 mL) was added DIPEA (3.65 g, 28.2 mmol, 3 equiv.). The mixture was stirred at 50 °C for 12 hours, then poured into water and extracted with EtOAc. The combined organic layers were washed with brine, dried over Na₂SO₄, filtered and concentrated. The crude product was purified by silica gel chromatography using a petroleum ether/ethyl acetate gradient as eluent to provide the title compound (1.25 g, 3.5 mmol, 37% yield) as yellow solid. LC-MS: m/z = 358.2 [M+H]⁺, ESI pos.

### Step 3: 3-Chloro-8-[(2, 4-dimethoxyphenyl)methyl]-6, 7-dihydropyridazino[4,3-b][1,4]oxazine

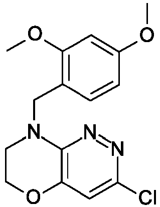

To a solution of Cs₂CO₃ (2.55 g, 7.82 mmol, 2 equiv.) and 2-(3,6-dichloropyridazin-4-yl)oxy-N-[(2,4-dimethoxyphenyl)methyl]ethanamine (1.4 g, 3.91 mmol, 1 equiv.) in toluene (40 mL) was added BINAP (340.7 mg, 0.55 mmol, 0.14 equiv.). The mixture was stirred at 110 °C for 2 hours, then diluted with DCM and filtered through a pad of celite. The filtrate was washed with brine. The organic layers were dried over Na₂SO₄, filtered and concentrated. The crude product was purified by silica column chromatography using a petroleum ether/ethyl acetate gradien (900 mg, 2.8 mmol, 72% yield) as yellow solid. LC-MS: m/z = 322.1 [M+H]⁺, ESI pos.

¹H NMR (400 MHz, CDCl₃-*d*) δ= 7.03 (d, J = 8.3 Hz, 1H), 6.65 (s, 1H), 6.53 - 6.43 (m, 2H), 4.40 (s, 2H), 4.39 - 4.35 (m, 2H), 3.82 (s, 6H), 3.49 - 3.44 (m, 2H).

### Step 4: 8-[(2,4-Dimethoxyphenyl)methyl]-3-methyl-6,7-dihydropyridazino[4,3-b][1,4]oxazine

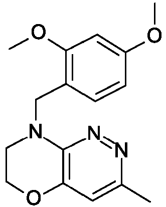

To a solution of trimethylboroxine (971.4 mg, 3.87 mmol, 1.5 equiv.) and 3-chloro-8-[(2,4-dimethoxyphenyl)methyl]-6,7-dihydropyridazino[4,3-b][1,4]oxazine (830.0 mg, 2.58 mmol, 1 equiv.) in 1,4-dioxane (15 mL) were added Pd(dppf)Cl₂ (189 mg, 0.26 mmol, 0.1 equiv.) and K₂CO₃ (713 mg, 5.16 mmol, 2 equiv.) under a nitrogen atmosphere. The mixture was stirred at 100 °C for 12 hours, then diluted with DMF (10mL) and MeOH (40mL). Thiourea on resin was added, and and stirring was continued for 3 hours. The mixture was filtered and concentrated. The crude product was purified by prep-HPLC using an 0.1% aqueous formic acid/acetonitrile gradient as eluent. The product containing fraction were lyophilized to give the title compound (600 mg, 1.99 mmol, 77% yield) as yellow gum. LC-MS: m/z = 302.1 [M+H]⁺, ESI pos.

### Step 5: 3-Methyl-7,8-dihydro-6H-pyridazino[4,3-b][1,4]oxazine

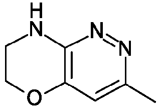

A solution of 3-chloro-8-[(2,4-dimethoxyphenyl)methyl]-6,7-dihydropyridazino[4,3-b][1,4]oxazine (550.0 mg, 1.71 mmol, 1 equiv.) in TFA (5.0 mL, 67.3 mmol, 39.5 equiv.) was stirred at 25 °C for 4 hours. The mixture was diluted with DCM, then basified by the addition of aqueous ammonia to bring the pH to 8 - 9. Then, the mixture was extracted with water. The aqueous layer was concentrated in vacuo. The residue was purified by prep-HPLC using a 10 mM aqueous NH₄CO₃ solution/acetonitrile gradient as eluent. The product-containing fractions were lyophilized to provide the title compound (88.8 mg, 0.59 mmol, 34% yield) as white solid. LC-MS: m/z = 152.0 [M+H]⁺, ESI pos.

¹H NMR (400 MHz, MeOH-d₄) δ= 6.54 (s, 1H), 4.41 - 4.34 (m, 2H), 3.50 - 3.42 (m, 2H), 2.36 (s, 3H).

### Step 6: 1-[3-Acetyl-6-[6-(3-methyl-6, 7-dihydropyridazino[4,3-b][1,4]oxazin-8-yl)imidazo[4,5-c]pyridin-3-yl]-2-pyridyl]-5-methyl-pyrazole-3-carbonitrile

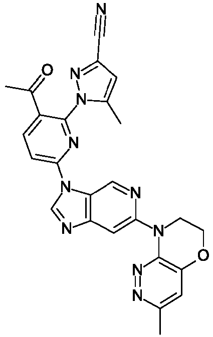

A suspension of 1-[3-acetyl-6-(6-chloroimidazo[4,5-c]pyridin-3-yl)-2-pyridyl]-5-methylpyrazole-3-carbonitrile (example 1, step 1; 100 mg, 0.24 mmol, 1.0 equiv.), 3-methyl-7,8-dihydro-6H-pyridazino[4,3-b][1,4]oxazine (36.0 mg, 0.24 mmol, 1.0 equiv.) and cesium carbonate (388.1 mg, 1.19 mmol, 5.0 equiv.) at RT in 1,4-dioxane (6.56 mL) was flushed with argon for 5 minutes. Then [tBuBrettPhos Pd(allyl)]OTf (18.6 mg, 0.024 mmol, 0.10 equiv.) was added. The reaction vial was sealed, and the mixture was heated to 80 °C for 2 hours. Again, [tBuBrettPhos Pd(allyl)]OTf (18.61 mg, 0.024 mmol, 0.10 eq) were added and heating to 80 °C was continued for 2 hours. The reaction mixture was cooled to RT, again flushed with argon and treated with [tBuBrettPhos Pd(allyl)]OTf (18.61 mg, 0.024 mmol, 0.10 eq) and stirred for 1 hour at 80 °C. The reaction mixture was concentrated. The crude product was purified by silica gel chromatography using a dichloromethane/MeOH gradient as eluent. The product-containing fractions were concentrated. The residue was triturated with diethylether. The solid was filtered and dried to provide the title compound (23 mg, 18%) as light brown solid. LC-MS: m/z = 493.2 [M+H]⁺, ESI pos.

### Example 7

### 1-[2-[3-(difluoromethyl)-5-methyl-pyrazol-1-yl]-6-[6-[(6-methylpyridazin-3-yl)amino]imidazo[4,5-c]pyridin-3-yl]-3-pyridyl]ethanol

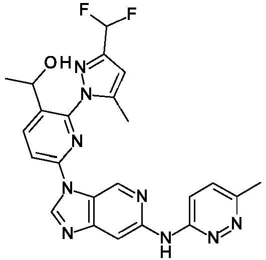

### Step 1: 1-[6-Chloro-2-[3-(difluoromethyl)-5-methyl-pyrazol-1-yl]-3-pyridyl]ethanone

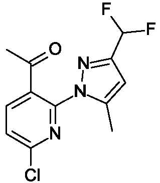

A mixture of 1-(6-chloro-2-fluoro-3-pyridyl)ethanone (1.0 g, 5.76 mmol, 1.0 equiv.), 3-(difluoromethyl)-5-methyl-1H-pyrazole (761.13 mg, 5.76 mmol, 1.0 equiv.) and DIPEA (2.22 g, 17.3 mmol, 3.0 equiv.) in DMSO (10 mL) was stirred at 100 °C for 12 hours. The crude product was purified by reversed phase chromatography using an acetonitrile/0.1% aqueous formic acid gradient as eluent and lyophilized to afford the title compound (800 mg, 2.8 mmol, 49% yield) as a grey solid. LC-MS: m/z = 286.1, [M+H]⁺, ESI pos.

### Step 2: 1-[2-[3-(Difluoromethyl)-5-methyl-pyrazol-1-yl]-6-[6-[(6-methylpyridazin-3-yl)amino[imidazo[4,5-c]pyridin-3-yl]-3-pyridyl]ethanone

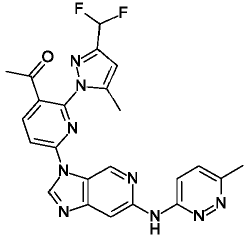

To a solution of N-(6-methylpyridazin-3-yl)-3H-imidazo[4,5-c]pyridin-6-amine (example 4, step 6; 230.0 mg, 0.71 mmol, 1.0 equiv.) in DMSO (5 mL) was added 1-[6-chloro-2-[3-(difluoromethyl)-5-methyl-pyrazol-1-yl]-3-pyridyl]ethanone (example 1, step 1; 203.3 mg, 0.71 mmol, 1.0 equiv.) and K₂CO₃ (196.7 mg, 1.42 mmol, 2.0 equiv.). The mixture was stirred at 70 °C for 12 hours, then diluted with water and extracted with EtOAc. The combined organic layers were washed with brine, dried over with anhydrous sodium sulfate and concentrated. The residue was purified twice by silica gel chromatography using a hexane/ethyl acetate gradient as eluent to give a mixture of regioisomers 1-[2-[3-(difluoromethyl)-5-methyl-pyrazol-1-yl]-6-[6-[(6-methylpyridazin-3-yl)amino]imidazo[4,5-c]pyridin-3-yl]-3-pyridyl]ethanone and 1-[2-[3-(difluoromethyl)-5-methyl-pyrazol-1-yl]-6-[6-[(6-methylpyridazin-3-yl)amino]imidazo[4,5-c]pyridin-1-yl]-3-pyridyl]ethanone (30.0 mg, 0.06 mmol, 9% yield) as a yellow solid. LC-MS: m/z = 476.2, [M+H]⁺, ESI pos.

This mixture was separated by SFC using a acetonitrile/MeOH + 0.1%NH3 as and eluent to obtain the title compound 1-[2-[3-(difluoromethyl)-5-methyl-pyrazol-1-yl]-6-[6-[(6-methylpyridazin-3-yl)amino]imidazo[4,5-c]pyridin-3-yl]-3-pyridyl]ethanone (10 mg, 32% yield) as a yellow solid. LC/MS: m/z = 476.1, [M+H]⁺, ESI pos.

¹H NMR (400 MHz, DMSO-d₆) δ = 9.99 (s, 1H), 9.33 (s, 1H), 9.22 (d, J = 0.7 Hz, 1H), 8.44 (d, J = 8.4 Hz, 1H), 8.34 (s, 1H), 8.29 (d, J = 8.4 Hz, 1H), 7.76 (d, J = 9.3 Hz, 1H), 7.41 (d, J = 9.2 Hz, 1H), 7.22 - 6.90 (m, 1H), 6.76 (s, 1H), 2.59 (s, 3H), 2.54 (br s, 3H), 2.03 (s, 3H).

In addition, the regioisomer 1-[2-[3-(difluoromethyl)-5-methyl-pyrazol-1-yl]-6-[6-[(6-methylpyridazin-3-yl)amino]imidazo[4,5-c]pyridin-1-yl]-3-pyridyl]ethanone (2 mg, 0.02 mmol, 7% yield) as a yellow solid. LC-MS: m/z = 476.1, [M+H]⁺, ESI pos.

### Step 3: 1-[2-[3-(difluoromethyl)-5-methyl-pyrazol-1-yl]-6-[6-[(6-methylpyridazin-3-yl)amino[imidazo[4,5-c]pyridin-3-yl]-3-pyridyl]ethanol

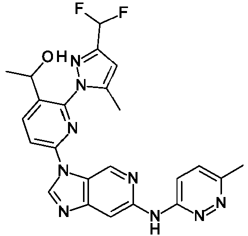

To a solution of 1-[2-[3-(difluoromethyl)-5-methyl-pyrazol-1-yl]-6-[6-[(6-methylpyridazin-3-yl)amino]imidazo[4,5-c]pyridin-3-yl]-3-pyridyl]ethanone (8.0 mg, 0.02 mmol, 1.0 equiv.) in MeOH (2 mL) was added a solution of NaBH₄ (3.18 mg, 0.08 mmol, 5.0 equiv.) in MeOH (0.5 mL) at -70 °C. The mixture was stirred at -70 °C for 0.5 hour. The reaction mixture was quenched by addition of aqueous ammonium chloride solution at 20 °C.

The crude product was purified reversed phase chromatography using an acetonitrile/0.1% aqueous formic acid gradient as eluent to give the title compound (1.2 mg, 14% yield) as a lyophilized white solid. LC-MS: m/z = 478.2, [M+H]⁺, ESI pos.

¹H NMR (400 MHz, MeOH-d₄) δ = 9.43 (s, 1H), 9.27 (s, 1H), 8.52 (d, J = 8.5 Hz, 1H), 8.20 (d, J = 8.5 Hz, 1H), 7.85 (s, 1H), 7.77 (s, 2H), 6.98 - 6.65 (m, 1H), 6.61 (s, 1H), 4.80 - 4.80 (m, 1H), 2.61 (s, 3H), 2.42 (s, 3H), 1.39 (d, J = 6.4 Hz, 3H).

### Example 8 - Phosphorylation assay SIK1-3:

In the presence of SIK2 (resp. SIK1 or SIK3) and ATP the CHK-peptide (KKKVSRSGLYRSPSMPENLNRPR with C-terminal arginine amide modification) were phosphorylated at one of the four feasible serine's. Only one phosphorylation is observed under the assay conditions. 60 nl of each compound dilution series (12 point; dilution factor 3, generally 30 µM to 170 pM) in DMSO were transferred by acoustic dispensing to the assay plate and 30 minutes pre-incubated (ambient temperature) after the addition of 5 µl SIK1 (5 nM) resp. 5 µl SIK2 (0.5 nM) or 7 µl SIK3 (1.5 nM) in assay-buffer (12.5 mM HEPES (pH 7.0), 10 mM magnesium acetate, 0.005% BSA). 10 µM CHK-peptide solution and 5 µl of 100 µM ATP for SIK1 & SIK2 resp. 3 µl for SIK3 in assay-buffer were added and incubated ambient for 45 minutes. 40 µl of 0.125% formic acid in water were added to quench the reaction. RapidFire (RF) Mass Spectrometry was utilized for data generation as described below. The multiple charged species (3-5 charges) for the phosphorylated and non-phosphorylated form measured by MRM (Multiple Reaction Monitoring; API5000 or 6500+) or EIC (Extracted Ion Current; QToF) were summed up and the ratio calculated (sum phosphorylated species / sum all species) for data evaluation. Normalization was performed by Genedata software based on the non-inhibition control DMSO and the commercially available SIK inhibitor @ 1µM YKL-05-099 (CAS number 1936529-65-5). The results of the assay are expressed in half-maximal inhibitory concentrations (IC50s) and are summarized below in Table 1.

### RapidFire Setup:

Samples were aspirated by vacuum for max. 600ms and loaded to C4-cartridge (Agilent; #G9203A) for 3000ms@1.5ml/min with 0.1% formic acid in water. Afterwards samples were transferred to the API5000 (API6500+) or QToF mass spectrometer for 4000ms@1.25ml/min with 90% acetonitrile; 10% water; 0.007% TFA; 0.093 formic acid. The cartridge was reconditioned for additional 500ms with 0.1% formic acid in water.

### MS-Setup Sciex API5000/API6500+:

All MS analyses using the following MS-setup in MRM mode: Electrospray positive; Ion Spray Voltage: 4000V; Temperature: 550 °C; Collision Gas: 5; Curtain Gas: 15; Gas 1: 40; Gas 2: 42; EP: 10. DP = declustering potential; CE = collision energy; CXP = cell exit potential.

| Name | Q1 (m/z) | Q3 (m/z) | Time(ms) | DP (V) | CE (V) | CXP (V) |
|---|---|---|---|---|---|---|
| CHK(4+) | 676.0 | 84.2 | 50 | 46 | 99 | 18 |
| CHK(5+) | 541.0 | 84.2 | 50 | 51 | 71 | 36 |
| pCHK(4+) | 696.0 | 84.2 | 50 | 66 | 105 | 18 |
| pCHK(5+) | 557.0 | 84.2 | 50 | 51 | 53 | 4 |

### MS-Setup Agilent QToF 6545

All MS analyses using the following MS-setup in Mode MS: Dual AJS Electrospray positive; VCap: 3000V; Drying & Sheath gas: 340 °C@8l/min; Nebulizer: 60psig; Nozzle Voltage: 2000V; Fragmentor: 130V; Skimmer: 35V; Oct1 RF Vpp: 700V; Ref masses on@5spectra/s

| Name | EIC (m/z) | Width |
|---|---|---|
| CHK(3+) | 900.8345 | 50ppm |
| CHK(4+) | 675.8789 | 50ppm |
| CHK(5+) | 540.9048 | 50ppm |
| pCHK(3+) | 927.4961 | 50ppm |
| pCHK(4+) | 695.8747 | 50ppm |

**Table 1: IC50 values for inhibition of SIK1, SIK2 and SIK3:**

| Example | SIK1 RF IC50 (uM) | SIK2 RF IC50 (uM) | SIK3 RF IC50 (uM) |
|---|---|---|---|
| 1 | 4.524 | 0.042 | 0.121 |
| 2 | 0.366 | 0.005 | 0.003 |
| 3 | 0.078 | 0.022 | 0.022 |
| 4 | 0.396 | 0.003 | 0.003 |
| 5 | 19.024 | 0.687 | 2.056 |
| 6 | 17.415 | 2.542 | 3.286 |
| 7 | 0.879 | 0.016 | 0.007 |

### Example A

Film coated tablets containing the following ingredients can be manufactured in a conventional manner:

| Ingredients | Per tablet | |
|---|---|---|
| **Kernel:** | | |
| Compound of formula (I) or a pharmaceutically acceptable salt thereof | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| **Film Coat:** | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titan dioxide | 0.8 mg | 1.6 mg |

The active ingredient is sieved and mixed with microcrystalline cellulose and the mixture is granulated with a solution of polyvinylpyrrolidone in water. The granulate is then mixed with sodium starch glycolate and magnesium stearate and compressed to yield kernels of 120 or 350 mg respectively. The kernels are lacquered with an aq. solution / suspension of the above mentioned film coat.

### Example B

Capsules containing the following ingredients can be manufactured in a conventional manner:

| Ingredients | Per capsule |
|---|---|
| Compound of formula (I) or a pharmaceutically acceptable salt thereof | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) or a pharmaceutically acceptable salt thereof | 3.0 mg |
| Polyethylene glycol 400 | 150.0 mg |
| Acetic acid | q.s. ad pH 5.0 |
| Water for injection solutions | ad 1.0 ml |

The active ingredient is dissolved in a mixture of Polyethylene glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

## Claims

1. A compound of formula (I) 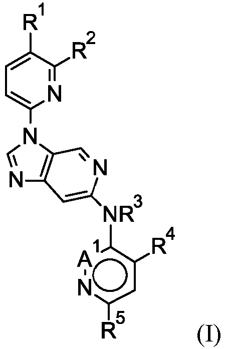 wherein
R¹ is hydroxyalkyl, alkylcarbonyl, cyclopropyl, haloalkyl or cyano;
R² is heteroaryl optionally substituted with one, two or three substituents independently selected from alkyl, haloalkyl, halogen, cyano, haloalkoxy and alkoxy;
R³ is hydrogen or alkyl;
R⁴ is hydrogen or alkyl; or R³ together with R⁴ and the atoms they are attached to, form a five- to seven-membered heterocycloalkyl or heteroaryl ring;
R⁵ is alkyl or haloalkyl; and
A¹ is -CH- or -N-;
or a pharmaceutically acceptable salt thereof.

2. A compound according to claim 1, wherein R¹ is hydroxyalkyl or alkylcarbonyl.

3. A compound according to claim 1 or 2, wherein R¹ is hydroxyethyl or methylcarbonyl.

4. A compound according to any one of claims 1 to 3, wherein R² is cyano, haloalkyl or haloalkoxy.

5. A compound according to any one of claims 1 to 4, wherein R² is cyano, difluoromethyl or difluoromethoxy.

6. A compound according to any one of claims 1 to 5, wherein R³ is hydrogen or methyl, and R⁴ is hydrogen; or R³ together with R⁴ and the atoms they are attached to, form a five- to seven-membered heterocycloalkyl or heteroaryl ring.

7. A compound according to any one of claims 1 to 6, wherein R³ is hydrogen or methyl, and R⁴ is hydrogen; or R³ together with R⁴ and the atoms they are attached to, form 3,4-dihydro-2H-1,4-oxazine.

8. A compound according to any one of claims 1 to 7, wherein R⁵ is alkyl, in particular wherein R⁵ is methyl.

9. A compound according to any one of claims 1 to 8, wherein A¹ is -N-.

10. A compound according to any one of claims 1 to 9, wherein A¹ is -CH-.

11. A compound of formula (I) according to any one of claims 1 to 10, selected from
1-[3-acetyl-6-[6-[(6-methylpyridazin-3-yl)amino]imidazo[4,5-c]pyridin-3-yl]-2-pyridyl]-5-methyl-pyrazole-3-carbonitrile;
1-[3-(1-hydroxyethyl)-6-[6-[(6-methylpyridazin-3-yl)amino]imidazo[4,5-c]pyridin-3-yl]-2-pyridyl]-5-methyl-pyrazole-3-carbonitrile;
1-[3-(1-hydroxyethyl)-6-[6-[(6-methyl-3-pyridyl)amino]imidazo[4,5-c]pyridin-3-yl]-2-pyridyl]-5-methyl-pyrazole-3-carbonitrile;
1-[6-[6-[(6-methylpyridazin-3-yl)amino]imidazo[4,5-c]pyridin-3-yl]-2-[3-methyl-1-(2,2,2-trifluoroethyl)pyrazol-4-yl]-3-pyridyl]ethanol;
1-[3-acetyl-6-[6-[methyl-(6-methylpyridazin-3-yl)amino]imidazo[4,5-c]pyridin-3-yl]-2-pyridyl]-5-methyl-pyrazole-3-carbonitrile;
1-[3-acetyl-6-[6-(3-methyl-6,7-dihydropyridazino[4,3-b][1,4]oxazin-8-yl)imidazo[4,5-c]pyridin-3-yl]-2-pyridyl]-5-methyl-pyrazole-3-carbonitrile; and
1-[2-[3-(difluoromethyl)-5-methyl-pyrazol-1-yl]-6-[6-[(6-methylpyridazin-3-yl)amino]imidazo[4,5-c]pyridin-3-yl]-3-pyridyl]ethanol;
or a pharmaceutically acceptable salt thereof.

12. A process for the preparation of a compound according to any one of claims 1 to 11, comprising one of the following steps:
(a) the reaction of a compound of formula (B1) 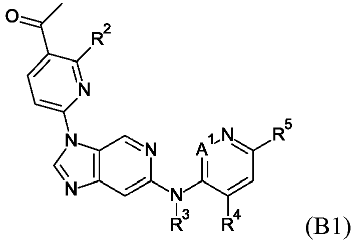 with a suitable reducing agent in presence of a suitable solvent,
(b) the reaction of a compound of formula (B2) 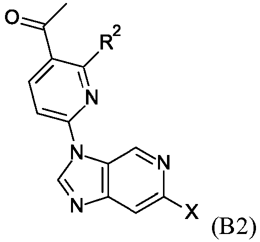 with a compound of formula (B3) 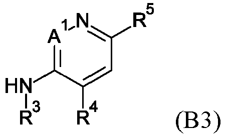 in presence of a suitable base, a suitable catalyst and a suitable solvent, or
(c) the reaction of a compound of formula (B4) 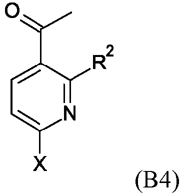 with a compound of formula (B5) 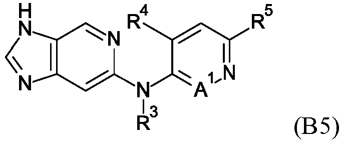 in presence of a suitable base,
wherein X is halogen; A¹, R², R³, R⁴ and R⁵ are as defined in any one of claims 1 to 9.

13. A compound of formula (I) according to any one of claims 1 to 11 or a pharmaceutcically acceptable salt thereof, for use as therapeutically active substance.

14. A pharmaceutical composition comprising a compound of formula (I) according to any one of claims 1 to 11 or a pharmaceutically acceptable salt thereof, and a therapeutically inert carrier.

15. A compound of formula (I) according to any one of claims 1 to 11 or a pharmaceutically acceptable salt thereof, for use in the treatment or prophylaxis of rheumatoid arthritis, juvenile rheumatoid arthritis, non-alcoholic steatohepatitis (NASH), primary sclerosing cholangitis, giant cell vasculitis, inflammatory bowel diseases (IBD), atherosclerosis, type 2 diabetes or glomerulonephritis.

## Patentansprüche

1. Verbindung der Formel (I) 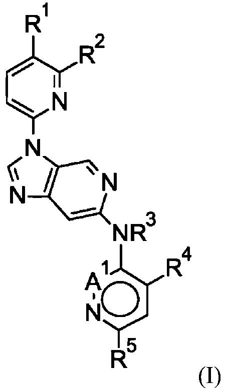 wobei
R¹ Hydroxyalkyl, Alkylcarbonyl, Cyclopropyl, Halogenalkyl oder Cyano ist;
R² Heteroaryl ist, das gegebenenfalls mit einem, zwei oder drei Substituenten substituiert ist, die unabhängig voneinander ausgewählt sind aus Alkyl, Halogenalkyl, Halogen, Cyano, Halogenalkoxy und Alkoxy;
R³ Wasserstoff oder Alkyl ist;
R⁴ Wasserstoff oder Alkyl ist; oder R³ zusammen mit R⁴ und den Atomen, an die sie gebunden sind, einen fünf- bis siebengliedrigen Heterocycloalkyl- oder Heteroarylring bildet;
R⁵ Alkyl oder Halogenalkyl ist; und
A¹ -CH- oder -N- ist;
oder ein pharmazeutisch verträgliches Salz davon.

2. Verbindung nach Anspruch 1, wobei R¹ Hydroxyalkyl oder Alkylcarbonyl ist.

3. Verbindung nach Anspruch 1 oder 2, wobei R¹ Hydroxyethyl oder Methylcarbonyl ist.

4. Verbindung nach einem der Ansprüche 1 bis 3, wobei R² Cyano, Halogenalkyl oder Halogenalkoxy ist.

5. Verbindung nach einem der Ansprüche 1 bis 4, wobei R² Cyano, Difluormethyl oder Difluormethoxy ist.

6. Verbindung nach einem der Ansprüche 1 bis 5, wobei R³ Wasserstoff oder Methyl ist und R⁴ Wasserstoff ist; oder R³ zusammen mit R⁴ und den Atomen, an die sie gebunden sind, einen fünf- bis siebengliedrigen Heterocycloalkyl- oder Heteroarylring bildet.

7. Verbindung nach einem der Ansprüche 1 bis 6, wobei R³ Wasserstoff oder Methyl ist und R⁴ Wasserstoff ist; oder R³ zusammen mit R⁴ und den Atomen, an die sie gebunden sind, 3,4-Dihydro-2H-1,4-oxazin bildet.

8. Verbindung nach einem der Ansprüche 1 bis 7, wobei R⁵ Alkyl ist, insbesondere wobei R⁵ Methyl ist.

9. Verbindung nach einem der Ansprüche 1 bis 8, wobei A¹ -N- ist.

10. Verbindung nach einem der Ansprüche 1 bis 9, wobei A¹ -CH- ist.

11. Verbindung der Formel (I) nach einem der Ansprüche 1 bis 10, ausgewählt aus
1-[3-Acetyl-6-[6-[(6-methylpyridazin-3-yl)amino]imidazo[4,5-c]pyridin-3-yl]-2-pyridyl]-5-methylpyrazol-3-carbonitril;
1-[3-(1-Hydroxyethyl)-6-[6-[(6-methylpyridazin-3-yl)amino]imidazo[4,5-c]pyridin-3-yl]-2-pyridyl]-5-methylpyrazol-3-carbonitril;
1-[3-(1-Hydroxyethyl)-6-[6-[(6-methyl-3-pyridyl)amino]imidazo[4,5-c]pyridin-3-yl]-2-pyridyl]-5-methylpyrazol-3-carbonitril;
1-[6-[6-[(6-Methylpyridazin-3-yl)amino]imidazo[4,5-c]pyridin-3-yl]-2-[3-methyl-1-(2,2,2-trifluorethyl)pyrazol-4-yl]-3-pyridyl]ethanol;
1-[3-Acetyl-6-[6-[methyl-(6-methylpyridazin-3-yl)amino]imidazo[4,5-c]pyridin-3-yl]-2-pyridyl]-5-methylpyrazol-3-carbonitril;
1-[3-Acetyl-6-[6-(3-methyl-6,7-dihydropyridazino[4,3-b][1,4]oxazin-8-yl)imidazo[4,5-c]pyridin-3-yl]-2-pyridyl]-5-methylpyrazol-3-carbonitril; und
1-[2-[3-(Difluormethyl)-5-methylpyrazol-1-yl]-6-[6-[(6-methylpyridazin-3-yl)amino]imidazo[4,5-c]pyridin-3-yl]-3-pyridyl]ethanol;
oder ein pharmazeutisch verträgliches Salz davon.

12. Verfahren zur Herstellung einer Verbindung nach einem der Ansprüche 1 bis 11, umfassend einen der folgenden Schritte:
(a) die Reaktion einer Verbindung der Formel (B1) 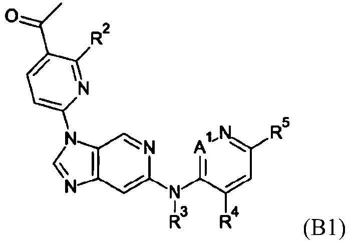 mit einem geeigneten Reduktionsmittel in Gegenwart eines geeigneten Lösungsmittels,
(b) die Reaktion einer Verbindung der Formel (B2) 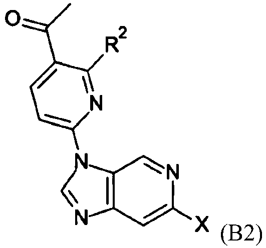 mit einer Verbindung der Formel (B3) 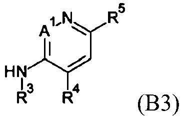 in Gegenwart einer geeigneten Base, eines geeigneten Katalysators und eines geeigneten Lösungsmittels, oder
(c) die Reaktion einer Verbindung der Formel (B4) 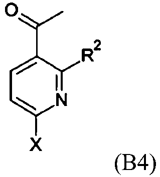 mit einer Verbindung der Formel (B5) 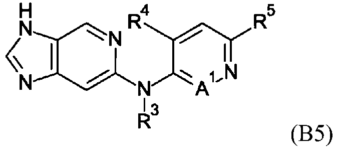 in Gegenwart einer geeigneten Base,
wobei X Halogen ist; A¹, R², R³, R⁴ und R⁵ wie in einem der Ansprüche 1 bis 9 definiert sind.

13. Verbindung der Formel (I) nach einem der Ansprüche 1 bis 11 oder ein pharmazeutisch verträgliches Salz davon zur Verwendung als therapeutisch wirksame Substanz.

14. Pharmazeutische Zusammensetzung, umfassend eine Verbindung der Formel (I) nach einem der Ansprüche 1 bis 11 oder ein pharmazeutisch verträgliches Salz davon und einen therapeutisch inerten Träger.

15. Verbindung der Formel (I) nach einem der Ansprüche 1 bis 11 oder ein pharmazeutisch verträgliches Salz davon zur Verwendung bei der Behandlung oder Prophylaxe von rheumatoider Arthritis, juveniler rheumatoider Arthritis, nichtalkoholischer Steatohepatitis (NASH), primärer sklerosierender Cholangitis, Riesenzellvaskulitis, entzündlichen Darmerkrankungen (IBD), Atherosklerose, Typ-2-Diabetes oder Glomerulonephritis.

## Revendications

1. Composé de formule (I) 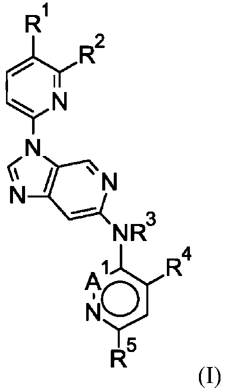 dans lequel
R¹ représente un hydroxyalkyle, un alkylcarbonyle, un cyclopropyle, un halogénoalkyle ou un cyano ;
R² représente un hétéroaryle éventuellement substitué par un, deux ou trois substituants indépendamment choisis parmi un alkyle, un halogénoalkyle, un halogène, un cyano, un halogénoalcoxy et un alcoxy ;
R³ représente un hydrogène ou un alkyle ;
R⁴ représente un hydrogène ou un alkyle ; ou R³ conjointement avec R⁴ et les atomes auxquels ils sont liés, forment un cycle hétérocycloalkyle ou hétéroaryle à cinq à sept chaînons ;
R⁵ représente un alkyle ou un halogénoalkyle ; et
A¹ représente -CH- ou -N- ;
ou un sel pharmaceutiquement acceptable de celui-ci.

2. Composé selon la revendication 1, dans lequel R¹ représente un hydroxyalkyle ou un alkylcarbonyle.

3. Composé selon la revendication 1 ou 2, dans lequel R¹ représente un hydroxyéthyle ou un méthylcarbonyle.

4. Composé selon l'une quelconque des revendications 1 à 3, dans lequel R² représente un cyano, un halogénoalkyle ou un halogénoalcoxy.

5. Composé selon l'une quelconque des revendications 1 à 4, dans lequel R² représente un cyano, un difluorométhyle ou un difluorométhoxy.

6. Composé selon l'une quelconque des revendications 1 à 5, dans lequel R³ représente un hydrogène ou un méthyle, et R⁴ représente un hydrogène ; ou R³ conjointement avec R⁴ et les atomes auxquels ils sont liés, forment un cycle hétérocycloalkyle ou hétéroaryle à cinq à sept chaînons.

7. Composé selon l'une quelconque des revendications 1 à 6, dans lequel R³ représente un hydrogène ou un méthyle, et R⁴ représente un hydrogène ; ou R³ conjointement avec R⁴ et les atomes auxquels ils sont liés, forment une 3,4-dihydro-2H-1,4-oxazine.

8. Composé selon l'une quelconque des revendications 1 à 7, dans lequel R⁵ représente un alkyle, en particulier dans lequel R⁵ représente un méthyle.

9. Composé selon l'une quelconque des revendications 1 à 8, dans lequel A¹ représente -N-.

10. Composé selon l'une quelconque des revendications 1 à 9, dans lequel A¹ représente -CH-.

11. Composé de formule (I) selon l'une quelconque des revendications 1 à 10, choisi parmi
le 1-[3-acétyl-6-[6-[(6-méthylpyridazin-3-yl)amino]imidazo[4,5-c]pyridin-3-yl]-2-pyridyl]-5-méthyl-pyrazole-3-carbonitrile ;
le 1-[3-(1-hydroxyéthyl)-6-[6-[(6-méthylpyridazin-3-yl)amino]imidazo[4,5-c]pyridin-3-yl]-2-pyridyl]-5-méthyl-pyrazole-3-carbonitrile ;
le 1-[3-(1-hydroxyéthyl)-6-[6-[(6-méthyl-3-pyridyl)amino]imidazo[4,5-c]pyridin-3-yl]-2-pyridyl]-5-méthyl-pyrazole-3-carbonitrile ;
le 1-[6-[6-[(6-méthylpyridazin-3-yl)amino]imidazo[4,5-c]pyridin-3-yl]-2-[3-méthyl-1-(2,2,2-trifluoroéthyl)pyrazol-4-yl]-3-pyridyl]éthanol ;
le 1-[3-acétyl-6-[6-[méthyl-(6-méthylpyridazin-3-yl)amino]imidazo[4,5-c]pyridin-3-yl]-2-pyridyl]-5-méthyl-pyrazole-3-carbonitrile ;
le 1-[3-acétyl-6-[6-(3-méthyl-6,7-dihydropyridazino[4,3-b][1,4]oxazin-8-yl)imidazo[4,5-c]pyridin-3-yl]-2-pyridyl]-5-méthyl-pyrazole-3-carbonitrile ; et
le 1-[2-[3-(difluorométhyl)-5-méthyl-pyrazol-1-yl]-6-[6-[(6-méthylpyridazin-3-yl)amino]imidazo[4,5-c]pyridin-3-yl]-3-pyridyl]éthanol ;
ou un sel pharmaceutiquement acceptable de ceux-ci.

12. Procédé pour la préparation d'un composé selon l'une quelconque des revendications 1 à 11, comprenant l'une des étapes suivantes :
(a) la réaction d'un composé de formule (B1) 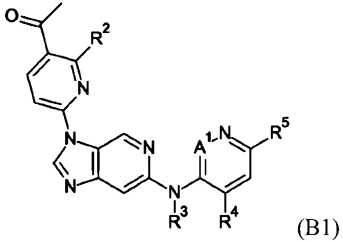 avec un agent réducteur approprié en présence d'un solvant approprié,
(b) la réaction d'un composé de formule (B2) 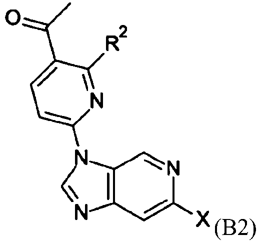 avec un composé de formule (B3) 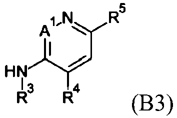 en présence d'une base appropriée, d'un catalyseur approprié et d'un solvant approprié, ou
(c) la réaction d'un composé de formule (B4) 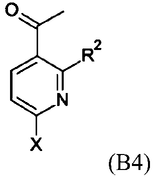 avec un composé de formule (B5) 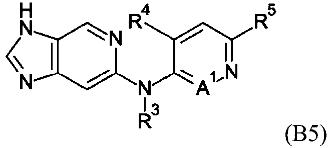 en présence d'une base appropriée,
dans lequel X représente un halogène ; A¹, R², R³, R⁴ et R⁵ sont tels que définis dans l'une quelconque des revendications 1 à 9.

13. Composé de formule (I) selon l'une quelconque des revendications 1 à 11 ou un sel pharmaceutiquement acceptable de celui-ci, pour une utilisation comme substance thérapeutiquement active.

14. Composition pharmaceutique comprenant un composé de formule (I) selon l'une quelconque des revendications 1 à 11 ou un sel pharmaceutiquement acceptable de celui-ci, et un véhicule thérapeutiquement inerte.

15. Composé de formule (I) selon l'une quelconque des revendications 1 à 11 ou un sel pharmaceutiquement acceptable de celui-ci, pour une utilisation dans le traitement ou la prophylaxie de la polyarthrite rhumatoïde, de la polyarthrite rhumatoïde juvénile, de la stéatohépatite non alcoolique (SHNA), de la cholangite sclérosante primitive, de la vascularite à cellules géantes, des maladies inflammatoires de l'intestin (MII), de l'athérosclérose, du diabète de type 2 ou de la glomérulonéphrite.
